(19) **Europäisches Patentamt / European Patent Office / Office européen des brevets**

(11) **EP 2 628 736 B1**

(12) **EUROPEAN PATENT SPECIFICATION**

(45) Date of publication and mention of the grant of the patent:
**07.10.2015 Bulletin 2015/41**

(51) Int Cl.:
*C07D 303/04* [(2006.01)]   *C07D 301/12* [(2006.01)]
*C07D 301/32* [(2006.01)]

(21) Application number: **11831927.6**

(22) Date of filing: **11.10.2011**

(86) International application number:
**PCT/CN2011/001704**

(87) International publication number:
**WO 2012/048530 (19.04.2012 Gazette 2012/16)**

(54) **Refining method for crude propylene oxide product and preparation method for propylene oxide**

Verfahren zur Raffination eines rohen Propylenprodukts und Verfahren zur Herstellung von Propylenoxid

Procédé de raffinage d'un brut d'oxyde de propylène et procédé de préparation d'oxyde de propylène

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.10.2010 CN 201010511514**
**11.10.2010 CN 201010511537**

(43) Date of publication of application:
**21.08.2013 Bulletin 2013/34**

(73) Proprietors:
- **China Petroleum & Chemical Corporation Beijing 100728 (CN)**
- **Hunan Changling Petrochemical Science and Technology Development Co., Ltd. Yueyang, Hunan 414012 (CN)**
- **Research Institute Of Petroleum Processing, Sinopec Beijing 100083 (CN)**

(72) Inventors:
- **LI, Hua**
  **Hunan 414012 (CN)**
- **LIN, Min**
  **Haidian**
  **Beijing 100083 (CN)**
- **GAO, Jizao**
  **Hunan 414012 (CN)**
- **WANG, Wei**
  **Hunan 414012 (CN)**
- **HE, Chijian**
  **Hunan 414012 (CN)**
- **WU, Xiaoju**
  **Hunan 414012 (CN)**
- **SHE, Xichun**
  **Hunan 414012 (CN)**
- **HE, Xiaojun**
  **Hunan 414012 (CN)**
- **CHEN, Qingling**
  **Hunan 414012 (CN)**

(74) Representative: **Hoffmann Eitle**
**Patent- und Rechtsanwälte PartmbB**
**Arabellastraße 30**
**81925 München (DE)**

(56) References cited:
**WO-A1-2004/092150    CN-A- 1 444 576**
**CN-A- 101 693 703    US-A- 3 881 996**

- **SAMIR I. ABU-EISHAH ET AL: "Design and control of a two-column azeotropic distillation system", INDUSTRIAL & ENGINEERING CHEMISTRY PROCESS DESIGN AND DEVELOPMENT, vol. 24, no. 1, 1 January 1985 (1985-01-01), pages 132-140, XP055101913, ISSN: 0196-4305, DOI: 10.1021/i200028a024**

**Description**

Technical Field

The present invention relates to a process for refining a crude propylene oxide product and a process for preparing propylene oxide including the refining process.

Background of the Invention

Currently, the propylene oxide production is mainly conducted worldwide by means of the hydrochlorin route method and the co-oxidation method. The hydrochlorin route method will be gradually eliminated due to the pollution problem. The co-oxidation method seems hardly to be developed to a large scale due to the limited use of its byproducts. Therefore, the production of propylene oxide is greatly limited by the production methods. In recent years, a new route for preparing propylene oxide comes out. According to this new route, propylene is epoxidated with an oxidant $H_2O_2$ under the titanium silicate catalysis to become propylene oxide. This route has the advantages such as the mild reaction condition and the environmentally friendly process without pollution, and therefore becomes a new green technology for producing propylene oxide.

The propylene epoxidation with $H_2O_2$ as oxidant and with the titanium silicate as catalyst to catalytically produce propylene oxide can be conducted in a fixed bed reactor or a fluidized bed reactor.

CN1671678A discloses an epoxidation method with two fixed bed reactors, wherein the first reactor is an isothermal fixed bed reactor, and the second reactor is a thermal insulation fixed bed reactor. The disadvantage of this method includes the conversion of $H_2O_2$ used in the reaction is not completely, and the unreacted $H_2O_2$ will decompose in the separation column to produce oxygen, which causes a safety concern and even an explosion when badly managed.

CN1449392A discloses a method for preparing an alkylene oxide with a peroxidized compound. In this method, the alkylene oxide is prepared by reacting an olefin and the peroxidized compound in at least two reactors in series (in each of the reactors, a part of catalyst is loaded) in the presence of catalyst and solvent. According to this method, the peroxidized compound is only supplied to the first reactor, and none of the fresh peroxidized compound is added to the subsequent one or more reactors, to each of which the unconsumed peroxidized compound from the preceding reactor is supplied to convert $H_2O_2$ completely in the reaction. The reactor used in this method is fixed bed reactor or fluidized bed reactor. According to this method, at least two reactors are used, preferably three reactors in series. The disadvantages of this method include if using two reactors at a minimum, the conversion of $H_2O_2$ is still not completely; if using more than two reactors in series, the device cost will increase remarkably, the reaction period of multiple reactors in series is long, and the uncontrollable factors in the reaction process are overmuch.

In the propylene epoxidation with $H_2O_2$ as oxidant and with the titanium silicate as catalyst to catalytically produce propylene oxide, methanol is generally used as solvent. Because the solvent of methanol is hardly consumed in the reaction, the reaction product is a mixture containing methanol, propylene oxide and the like. Since methanol and propylene oxide can form an azeotrope, the propylene oxide product obtained by separation after refining the crude propylene oxide product has a low purity, and the separation effect is impaired. In the method disclosed by US6500311B1, the mixture containing methanol and propylene oxide is separated by a liquid-liquid extraction with water and n-octane as extractant. Due to the introduction of n-octane as extractant into the separation system, there is a problem of separating and recovering n-octane, which results in a complex separation process.

WO 2004/092150 discloses a method for producing propylene oxide comprising separating propylene oxide from methanol by distillation.

J 15 Summary of the Invention

An object of the present invention is to address the problems in the prior art that the process for refining the crude propylene oxide product has an undesirable separation effect and cannot effectively separate methanol from propylene oxide and the obtained propylene oxide has a low purity, and to provide a novel process for refining a crude propylene oxide product, which process can effectively separate propylene oxide from methanol to produce a propylene oxide product having a relatively high purity, and a process for preparing propylene oxide including the refining process.

The present invention provides a process for refining a crude propylene oxide product, wherein said crude propylene oxide product is a mixture containing propylene oxide and methanol, wherein said process comprises:

(1) in a first azeotropic rectification condition, a crude propylene oxide product is fed to a first azeotropic rectification column to conduct an azeotropic rectification, said first azeotropic rectification condition is such a condition that a majority of propylene oxide and a part of methanol in a stream fed to said first azeotropic rectification column are discharged from the column top in an azeotrope form, and a residual part of methanol and a small amount of propylene oxide are discharged from the column bottom, wherein said residual part of methanol discharged from the column bottom of said first azeotropic rectification column has a purity of 50-99wt%, an effluent from the column bottom of said first azeotropic rectification column has a propylene oxide content of less than 5wt%;

(2) in a second azeotropic rectification condition, an azeotrope discharged from the column top of said first azeotropic rectification column in the step (1) is fed to a second azeotropic rectification column to conduct an azeotropic rectification, said second azeotropic rectification condition is such a condition that a majority of methanol and a part of propylene oxide in a mixture fed to said second azeotropic rectification column are discharged from the column top of said second azeotropic rectification column in an azeotrope form, a residual part of propylene oxide and a small amount of methanol are discharged from the column bottom of said second azeotropic rectification column, wherein said residual part of propylene oxide discharged from the column bottom of said second azeotropic rectification column has a purity of more than or equal to 95wt%, an effluent from the column bottom of said second azeotropic rectification column has a methanol content of less than 5wt%;

(3) optionally, the azeotrope discharged from the column top of said second azeotropic rectification column in the step (2) is partly or totally returned to said first azeotropic rectification column used in the step (1) to conduct an azeotropic rectification together with the crude propylene oxide product,

wherein said first azeotropic rectification condition and said second azeotropic rectification condition include: the pressure at the column top of said first azeotropic rectification column is 0.2-0.8MPa, and the pressure at the column top of said second azeotropic rectification column is 0.9-1.5MPa,
for said first azeotropic rectification column: the temperature at the column bottom is 100-140°C; the column plate number is 20-45, the refluxing ratio is 2-5:1, the feeding location is below a location that is away from the outlet of the column top by 1/4 of the distance from the outlet of the column bottom to the outlet of the column top and above a location that is away from the outlet of the column bottom by 1/4 of the distance from the outlet of the column bottom to the outlet of the column top, and is between the outlet of the column bottom and the outlet of the column top;
for said second azeotropic rectification column: the temperature at the column bottom is 110-130°C; the column plate number is 15-25, the refluxing ratio is 0.8-3:1, the feeding location is below a location that is away from the outlet of the column top by 1/4 of the distance from the outlet of the column bottom to the outlet of the column top and above a location that is away from the outlet of the column bottom by 1/4 of the distance from the outlet of the column bottom to the outlet of the column top.

The present invention also provides a process for preparing propylene oxide, which process comprises: propylene and $H_2O_2$ are contacted and reacted under a propylene epoxidation reaction condition in the presence of a solid catalyst and methanol to produce a crude propylene oxide product, and the resulting crude propylene oxide product is refined, wherein the resulting crude propylene oxide product is refined with the process for refining a crude propylene oxide product as provided in the present invention.

It is found by the inventors of the present invention that in the propylene epoxidation with $H_2O_2$ as oxidant and with the titanium silicate as catalyst to catalytically produce propylene oxide, methanol is generally used as solvent; because the solvent of methanol is hardly consumed in the reaction, the reaction product is a mixture containing methanol, propylene oxide and the like; since methanol and propylene oxide can form an azeotrope, therefore the propylene oxide product obtained by separation with a simple propylene oxide separation column still contains methanol, the propylene oxide product has a relatively low purity, and the product quality is impaired.

The inventors of the present invention skillfully utilize the characteristic that methanol and propylene oxide can form azeotropes, and accomplish the effective separation of propylene oxide and methanol by the following steps: firstly under an azeotropic rectification condition, an azeotrope having an azeotropic composition is formed from the crude propylene oxide product (i.e., a mixture containing methanol and propylene oxide) in a first azeotropic rectification column, and separated in said first azeotropic rectification column; then under another azeotropic rectification condition, the azeotrope discharged from the column top of said first azeotropic rectification column is fed to a second azeotropic rectification column to form an azeotrope having an azeotropic composition, which is separated in said azeotropic rectification column; and the azeotrope having an azeotropic composition discharged from the column top of said second azeotropic rectification column is recycled to said first azeotropic rectification column to conduct an azeotropic rectification along with the crude propylene oxide product, and the propylene oxide product having a relatively high purity is directly discharged from the column bottom of said second azeotropic rectification column.

The inventors of the present invention utilize the characteristic that the azeotropes formed from methanol and propylene oxide under different azeotropic rectification conditions have different azeotropic compositions, wherein in the first azeotropic separation, a reaction product containing a majority of propylene oxide can be discharged from the column top of said first azeotropic rectification column (at this time, a majority of methanol in the reaction product can be discharged from the column bottom of said first azeotropic rectification column); after the mixture discharged from the column top of said first azeotropic rectification column is fed to said second azeotropic rectification column, the azeotropic rectification condition can be changed in the second azeotropic separation so as to obtain an azeotrope having a different azeotropic composition from that of the azeotrope obtained from the column top of said first azeotropic rectification column; a majority of methanol contained in the mixture coming into said second azeotropic rectification column is discharged in an azeotrope form from the column top of said second azeotropic rectification column; and a propylene oxide product with high purity (i.e., having a relatively low content of methanol) can be successfully discharged from the column bottom of said second azeotropic rectification column. Therefore, the effective separation of propylene oxide and methanol can be accomplished with the refining process of the present invention, and the purity of the propylene oxide product can be remarkably improved.

Furthermore, it is found by the inventors of the present invention that in the production of propylene oxide from propylene and $H_2O_2$ with a titanium silicate as catalyst and with methanol as solvent, the conversion of propylene, the selectivity for propylene oxide and the safe threshold of the production system can be effectively improved, and the purity of propylene oxide can be economically and effectively increased by controlling the ratio of propylene and $H_2O_2$ in the reaction materials, using a combination of a fixed bed reactor and/or a moving bed reactor + a complete mixing reactor (such as a slurry bed reactor), separating $H_2O_2$ from the reaction product of the fixed bed reactor and/or the moving bed reactor and adding propylene to the reaction stream containing said separated $H_2O_2$, and using two azeotropic rectification columns as mentioned above to separate the crude reaction product.

Therefore, the present invention provides the following Schemes:

Scheme 1:

A process for refining a crude propylene oxide product, wherein said crude propylene oxide product is a mixture containing propylene oxide and methanol, which is characterized that said process comprises:

(1) in a first azeotropic rectification condition, a crude propylene oxide product is fed to a first azeotropic rectification column to conduct an azeotropic rectification, said first azeotropic rectification condition is such a condition that a majority of propylene oxide and a part of methanol in a stream fed to said first azeotropic rectification column are discharged from the column top in an azeotrope form, and a residual part of methanol and a small amount of propylene oxide are discharged from the column bottom, wherein said residual part of methanol discharged from the column bottom of said first azeotropic rectification column has a purity of 50-99wt%, an effluent from the column bottom of said first azeotropic rectification column has a propylene oxide content of less than 5wt%;

(2) in a second azeotropic rectification condition, an azeotrope discharged from the column top of said first azeotropic rectification column in the step (1) is fed to a second azeotropic rectification column to conduct an azeotropic rectification, said second azeotropic rectification condition is such a condition that a majority of methanol and a part of propylene oxide in a mixture fed to said second azeotropic rectification column are discharged from the column top of said second azeotropic rectification column in an azeotrope form, a residual part of propylene oxide and a small amount of methanol are discharged from the column bottom of said second azeotropic rectification column, wherein said residual part of propylene oxide discharged from the column bottom of said second azeotropic rectification column has a purity of more than or equal to 95wt%, an effluent from the column bottom of said second azeotropic rectification column has a methanol content of less than 5wt%;

(3) optionally, the azeotrope discharged from the column top of said second azeotropic rectification column in the step (2) is partly or totally returned to said first azeotropic rectification column used in the step (1) to conduct an azeotropic rectification together with the crude propylene oxide product, wherein said first azeotropic rectification condition and said second azeotropic rectification condition include: the pressure at the column top of said first azeotropic rectification column is 0.2-0.8MPa, and the pressure at the column top of said second azeotropic rectification column is 0.9-1.5MPa,

for said first azeotropic rectification column: the temperature at the column bottom is 100-140°C; the column plate number is 20-45, the refluxing ratio is 2-5:1, the feeding location is below a location that is away from the outlet of the column top by 1/4 of the distance from the outlet of the column bottom to the outlet of the column top and above a location that is away from the outlet of the column bottom by 1/4 of the distance from the outlet of the column bottom to the outlet of the column top, and is between the outlet of the column bottom and the outlet of the column top;

for said second azeotropic rectification column: the temperature at the column bottom is 110-130°C; the column plate number is 15-25, the refluxing ratio is 0.8-3:1, the feeding location is below a location that is away from the outlet of the column top by 1/4 of the distance from the outlet of the column bottom to the outlet of the column top and above a location that is away from the outlet of the column

bottom by 1/4 of the distance from the outlet of the column bottom to the outlet of the column top. Herein, in the expression "said first azeotropic rectification condition is such a condition that a majority of propylene oxide and a part of methanol in a stream fed to said first azeotropic rectification column are discharged from the column top in an azeotrope form, and a residual part of methanol and a small amount of propylene oxide are discharged from the column bottom", the term "a majority of propylene oxide" means more than 90%, for example more than 95% of propylene oxide, while the term "a part of methanol" means less than 30%, for example less than 10% or 5% of methanol.

Herein, in the expression "said second azeotropic rectification condition is such a condition that a majority of methanol and a part of propylene oxide in a mixture fed to said second azeotropic rectification column are discharged from the column top of said second azeotropic rectification column in an azeotrope form, a residual part of propylene oxide and a small amount of methanol are discharged from the column bottom of said second azeotropic rectification column", the term "a majority of methanol" means more than 90%, for example more than 95% of methanol, while the term "a part of propylene oxide" means 30-70%, for example 40-60% or 45-55% of propylene oxide.

Scheme 2:

The process according to Scheme 1, wherein in said crude propylene oxide product, the content of propylene oxide is 5-99.5wt%, the content of methanol is 0.5-95wt%, based on the total weight of the crude propylene oxide product containing methanol and propylene oxide.
In an embodiment, the crude propylene oxide product is consisted of: propylene oxide, methanol, $H_2O_2$, water, propylene glycol, propylene, and the other substance, wherein
the content of propylene oxide is 5-30wt%,
the content of methanol is 30-90wt%,
the content of $H_2O_2$ is less than 2wt%,
the content of water is 5-70wt%,
the content of propylene glycol is less than 10wt%,
the content of propylene is less than 5wt%, and
the content of the other substance is less than 2wt%,
wherein the amount of the crude propylene oxide product is 100wt%.
In an embodiment, the crude propylene oxide product is consisted of: propylene oxide, methanol, $H_2O_2$, water, propylene glycol, propylene, and the other substance, wherein
the content of propylene oxide is 10-20wt%,
the content of methanol is 40-80wt%,
the content of $H_2O_2$ is less than 1wt%,
the content of water is 10-50wt%,
the content of propylene glycol is less than 5wt%,
the content of propylene is less than 3wt%, and
the content of the other substance is less than 1wt%,
wherein the amount of the crude propylene oxide product is 100wt%.
In an embodiment, the crude propylene oxide product is consisted of: propylene oxide, methanol, $H_2O_2$, water, propylene glycol, propylene, and the other substance, wherein
the content of propylene oxide is 12-15wt%,
the content of methanol is 55-65wt%,
the content of $H_2O_2$ is less than 0.5 wt%,
the content of water is 20-30wt%,
the content of propylene glycol is less than 2wt%,
the content of propylene is less than 1wt%, and
the content of the other substance is less than 0.5wt%,
wherein the amount of the crude propylene oxide product is 100wt%.

Scheme 3:

The process according to Scheme 1, wherein an effluent from the column bottom of said first azeotropic rectification column has a propylene oxide content of less than 3wt%, and an effluent from the column bottom of said second azeotropic rectification column has a methanol content of less than 3wt%.

Scheme 4:

The process according to Scheme 1, wherein said first azeotropic rectification condition and said second azeotropic rectification condition are such conditions that the content of methanol in the azeotrope discharged from the column top of said first azeotropic rectification column is less than the content of methanol in the azeotrope discharged from the column top of said second azeotropic rectification column.

Scheme 5:

The process according to Scheme 1, wherein in the step (1), in the mixture discharged from the column top of said first azeotropic rectification column, the weight concentration of propylene oxide is 90-98wt%, and the weight concentration of methanol is 2-10wt%; in the step (2), in the mixture discharged from the column top of said second azeotropic rectification column, the weight concentration of propylene oxide is 85wt% to less than 98wt%, and the weight concentration of methanol is more than 2wt% to 15wt%.

Scheme 6:

The process according to Scheme 1, wherein said first azeotropic rectification condition and said second azeotropic rectification condition further include: the temperature at the column top of said second azeotropic rectification column is more than the temperature at the column top of said first azeotropic rectification column.

Scheme 7:

The process according to Scheme 1, wherein said first azeotropic rectification condition and said second azeotropic rectification condition include: the temperature at the column top of said first azeotropic rectification column is 60-107°C, the temperature at the column top of said second azeotropic rectification column is 110-125°C.

Scheme 8:

A process for preparing propylene oxide, which process comprises propylene and $H_2O_2$ are contacted and reacted under a propylene epoxidation reaction condition in the presence of a solid catalyst and methanol and optionally the residual propylene after the reaction is removed to produce a crude propylene oxide product, and the resulting crude propylene oxide product is refined, which is characterized that, the resulting crude propylene oxide product is refined according to the process of any one of Schemes 1-7.

Scheme 9:

A preparation process according to Scheme 8, wherein the reaction of propylene and $H_2O_2$ in the presence of a solid catalyst and methanol comprises a mixed stream containing methanol, propylene and $H_2O_2$ is charged successively into a first reactor and a second reactor, wherein said first reactor is a fixed bed reactor or a moving bed reactor, and said second reactor is a slurry bed reactor,
in said first reactor, said propylene epoxidation reaction is conducted so that the conversion rate of $H_2O_2$ reaches 50%-95%; in said second reactor, said propylene epoxidation reaction is conducted so that the total conversion rate of $H_2O_2$ reaches 98% or more, with a proviso that said process for preparing propylene oxide has a selectivity for propylene oxide of 90% or more.

Scheme 10:

A preparation process according to Scheme 9, wherein
in said fixed bed reactor, said propylene epoxidation reaction condition includes: the temperature is 30-90°C, the pressure is 0.5-4.5MPa, and the liquid volume hourly space velocity of said mixed stream is 1-15h$^{-1}$;
in said moving bed reactor, said propylene epoxidation reaction condition includes: the temperature is 30-90°C, the pressure is 0.5-4.5MPa, the reaction time is 0.2-10 hrs, with respect to 100 parts by weight of the mixed stream coming into said moving bed reactor and containing methanol, propylene and $H_2O_2$, the used amount of said solid catalyst is 3-10 parts by weight;
in said slurry bed reactor, said propylene epoxidation reaction condition includes: the temperature is 30-90°C, the pressure is 0.5-4.5MPa, the reaction time is 0.2-10 hrs, with respect to 100 parts by weight of the stream coming from said fixed bed reactor or moving bed reactor and coming into slurry bed reactor, the used amount of said solid catalyst is 3-10 parts by weight.

Scheme 11:

The process according to Scheme 9, wherein said process further comprises before the resulting reaction product obtained from the first reactor comes into said second reactor, the resulting reaction product obtained from the first reactor is subjected to a separation to obtain a first stream free of $H_2O_2$ and a second stream containing the unreacted $H_2O_2$, wherein said first stream free of $H_2O_2$ contains propylene and propylene oxide, and said second stream containing the unreacted $H_2O_2$ further contains the solvent and water; propylene is added to said second stream, and said second stream to which propylene has been added is charged into said second reactor,
optionally, the weight ratio of propylene added to said second stream to $H_2O_2$ in said second stream is 1-3:1, or 1-2:1.

Scheme 12:

The process according to Scheme 9, wherein said process further comprises before the resulting reaction product obtained from the first reactor comes into said second reactor, propylene is supplemented to the reaction product obtained in said first reactor, the weight ratio of the supplemented propylene to $H_2O_2$ in the reaction product obtained from said first reactor is 1-3:1, or 1-2:1.

Scheme 13:

The process according to Scheme 9, wherein the molar ratio of methanol, propylene and $H_2O_2$ in said mixed stream is 4-15:0.5-5:1.

Scheme 14:

The process according to Scheme 9, wherein said solid catalyst is a titanium silicate.

Scheme 15:

The process according to Scheme 9, wherein the residual propylene after reaction is removed so that in the resulting crude propylene oxide product, the content of propylene is less than 5wt%, e.g. 3wt%, or 1wt%, based on the weight of the crude propylene oxide product.

Description of the Drawings

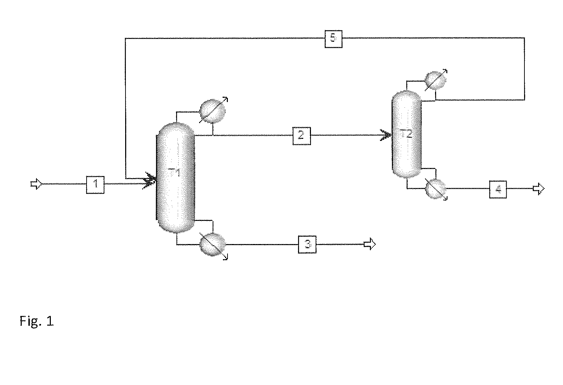

Figure 1 is a process chart of a process for refining a crude propylene oxide product according to the present invention.

Best Modes of Carrying out the Invention

In an aspect of the invention, the present invention provides a process for refining a crude propylene oxide product, wherein said crude propylene oxide product is a mixture containing propylene oxide and methanol. Said process for refining a crude propylene oxide product comprises:

(1) in a first azeotropic rectification condition, a crude propylene oxide product is fed to a first azeotropic rectification column to conduct an azeotropic rectification, said first azeotropic rectification condition is such a condition that a majority of propylene oxide and a part of methanol in a stream fed to said first azeotropic rectification column are discharged from the column top in an azeotrope form, and a residual part of methanol and a small amount of propylene oxide are discharged from the column bottom, wherein said residual part of methanol discharged from the column bottom of said first azeotropic rectification column has a purity of 50-99wt%, an effluent from the column bottom of said first azeotropic rectification column has a propylene oxide content of less than 5wt%;

(2) in a second azeotropic rectification condition, an azeotrope discharged from the column top of said first azeotropic rectification column in the step (1) is fed to a second azeotropic rectification column to conduct an azeotropic rectification, said second azeotropic rectification condition is such a condition that a majority of methanol and a part of propylene oxide in a mixture fed to said second azeotropic rectification column are discharged from the column top of said second azeotropic rectification column in an azeotrope form, a residual part of propylene oxide and a small amount of methanol are discharged from the column bottom of said second azeotropic rectification column, wherein said residual part of propylene oxide discharged from the column bottom of said second azeotropic rectification column has a purity of more than or equal to 95wt%, an effluent from the column bottom of said second azeotropic rectification column has a methanol content of less than 5wt%;

(3) optionally, the azeotrope discharged from the column top of said second azeotropic rectification column in the step (2) is partly or totally returned to said first azeotropic rectification column used in the step (1) to conduct an azeotropic rectification together with the crude propylene oxide product, wherein said first azeotropic rectification condition and said second azeotropic rectification condition include: the pressure at the column top of said first azeotropic rectification column is 0.2-0.8MPa, and the pressure at the column top of said second azeotropic rectification column is 0.9-1.5MPa,

for said first azeotropic rectification column: the temperature at the column bottom is 100-140°C; the column plate number is 20-45, the refluxing ratio is 2-5:1, the feeding location is below a location that is away from the outlet of the column top by 1/4 of the distance from the outlet of the column bottom to the outlet of the column top and above a location that is away from the outlet of the column bottom by 1/4 of the distance from the outlet of the column bottom to the outlet of the column top, and is between the outlet of the column bottom and the outlet of the column top;

for said second azeotropic rectification column: the temperature at the column bottom is 110-130°C; the column plate number is 15-25, the refluxing ratio is 0.8-3:1, the feeding location is below a location that is away from the outlet of the column top by 1/4 of the distance from the outlet of the column bottom to the outlet of the column top and above a location that is away from the outlet of the column bottom by 1/4 of the distance from the outlet of the column bottom to the outlet of the column top.

According to the present process for refining a crude propylene oxide product, said crude propylene oxide product generally has a propylene oxide content of 5-99.5wt%, and a methanol content of 0.5-95wt%, based on the total weight of the crude propylene oxide product containing methanol and propylene oxide; preferably, said crude propylene oxide product generally has a propylene oxide content of 10-95wt%, and a methanol content of 5-90wt%, based on the total weight of the crude propylene oxide product containing methanol and propylene oxide. Besides propylene oxide and methanol, said crude propylene oxide product generally contains other substances, such as those carried inevitably along with the preparation of the crude propylene oxide product, e.g. water and the unreacted starting materials such as propylene. In addition, in the process of refining the above-mentioned crude propylene oxide product, these substances are also inevitably present in the azeotropes discharged from the column tops of said first azeotropic rectification column and said second azeotropic rectification column and in the mixtures discharged from the column bottoms, with a proviso that the kinds or the contents of these substances have no negative effect on forming an azeotrope from methanol and propylene oxide.

According to the present process for refining a crude propylene oxide product, said azeotropic rectification operation conditions can be determined according to azeotropic compositions of azeotropes formed under different conditions from components in the crude propylene oxide product to be refined (i.e. the mixture containing propylene oxide and methanol). Preferably, in order to utilize the characteristic that the azeotropes formed under different azeotropic rectification conditions from methanol and propylene oxide have different azeotropic compositions, in the first azeotropic rectification, a refined reaction product containing a majority of propylene oxide can be discharged from the column top of said first azeotropic rectification column (at this time, a majority of methanol in the crude reaction product can be discharged from the column bottom of said first azeotropic rectification column); after the mixture from the column top of said first azeotropic rectification column is fed to said second azeotropic rectification column, in the second azeotropic rectification, the azeotropic rectification condition is changed so as to obtain an azeotrope that has an azeotropic composition different from the azeotrope from the column top of said first azeotropic rectification column, and therefore a majority of methanol contained in the mixture coming into said second azeotropic rectification column can be discharged from the column top of said second azeotropic rectification column in an azeotrope form, and a propylene oxide product of high purity (i.e. having a low content of methanol) can be successivefully discharged from the column bottom of said second azeotropic rectification column. Said first azeotropic rectification condition and said second azeotropic rectification condition are such conditions that the content of methanol in the azeotrope discharged from the column top of said first azeotropic rectification column is less than the content of methanol in the azeotrope discharged from the column top of said second azeotropic rectification column.

Preferably, in the step (1), the mixture discharged from the column top of said first azeotropic rectification column has a certain azeotropic composition under the first azeotropic rectification condition. For example, in the mixture discharged from the column top of said first azeotropic rectification column, the weight concentration of propylene oxide is 90-98wt%, and the weight concentration of methanol is 2-10wt%. In the step (2), the mixture discharged from the column top of said second azeotropic rectification column has a certain azeotropic composition under said second azeotropic rectification condition. For example, in the mixture discharged from the column top of said second azeotropic rectification column, the weight concentration of propylene oxide can be 85wt% to less than 98wt%, and the weight concentration of methanol can be more than 2wt% to 15wt%. Preferably, in the step (1), in the mixture discharged from the column top of said first azeotropic rectification column, the weight concentration of propylene oxide is 90-96wt%, and the weight concentration of methanol is 4-10wt%; and in the step (2), in the mixture discharged from the column top of said second azeotropic rectification column, the weight concentration of propylene oxide is 88-95wt%, and the weight concentration of methanol is 5-12wt%.

According to the present process for refining a crude propylene oxide product, the column top pressures of two azeotropic rectification columns are controlled so that the pressure at the column top of said second azeotropic rectification column is more than the pressure at the column top of said first azeotropic rectification column to achieve the different azeotropic compositions, i.e., the content of methanol in the azeotrope discharged from the column top of said first azeotropic rectification column is less than the content of methanol in the azeotrope discharged from the column top of said second azeotropic rectification column. Said first azeotropic rectification condition further includes the pressure at the column top of said first azeotropic rectification column is 0.2-0.8MPa, preferably 0.2-0.5MPa; and said second azeotropic rectification condition further includes the pressure at the column top of said second azeotropic rectification column is 0.9-1.5MPa, preferably 0.9-1.2MPa.

According to the present process for refining a crude propylene oxide product, the column top temperatures of two azeotropic rectification columns are controlled so that the temperature at the column top of said second azeotropic rectification column is more than the temperature at the column top of said first azeotropic rectification column to achieve the different azeotropic compositions, i.e., the content of methanol in the azeotrope discharged from the column top of said first azeotropic rectification column is less than the content of methanol in the azeotrope discharged from the column top of said second azeotropic rectification column. Preferably, said first azeotropic rectification condition further concludes the temperature at the column top of said first azeotropic rectification column is 60-107°C, preferably 60-95°C; and said second azeotropic rectification condition further concludes the temperature at the column top of said second azeotropic rectification column is 110-125°C, preferably 110-120°C.

According to the present process for refining a crude propylene oxide product, said first azeotropic rectification condition can further conclude the temperature at the column bottom of said first azeotropic rectification column is 100-140°C, preferably 100-130°C; and the refluxing ratio is 2-5:1. Said second azeotropic rectification condition can further conclude the temperature at the column bottom of said second azeotropic rectification column is 110-130°C, preferably 110-120°C; and the refluxing ratio is 0.8-3:1.

According to the present process for refining a crude propylene oxide product, said first azeotropic rectification columns and said second azeotropic rectification column can be a packed column, a plate column, or a combination thereof.

The packings charged in said packed column can be various packings well known to a person skilled in the art. For example, the packing can be selected from one or more of Raschig ring, Pall ring, cascade ring, saddle ring (Berl saddle, rectangular saddle), θ network ring, cannon ring, sheet corrugated structured packing and gauze structured packing.

In said plate column, one or more of bubble cap, sieve tray, oblique hole, and floating valve can be provided.

In order to achieve a desirable separation effect, said first azeotropic rectification column and said second azeotropic rectification column preferably have a certain column plate number. For example, the column plate number of said first azeotropic rectification column is 20-45; and the column plate number of said second azeotropic rectification column is 15-25.

According to the present process for refining a crude propylene oxide product, said first azeotropic rectification column and said second azeotropic rectification column can be in form of various azeotropic rectification columns well known to a person skilled in the art. For example, said azeotropic rectification columns can be composed of column body, column base, column base reboiler, column top condenser and column top reflux drum (separate phases). Preferably, on the side in the column lower part (any location below the feeding inlet and above the outlet of the column bottom) of said second azeotropic rectification column, at least one side outlet can be further provided to discharge the pure propylene oxide at the different side locations and avoid the product contamination at the column base. The number of said side outlet can be determined as demanded, preferably 1-5. The proportion of the discharging amount at the side outlet can be varied in a range of 0-100%. That is to say, it can be adjusted between all being discharged at the column base and all being discharged at the side outlet.

According to the present process for refining a crude propylene oxide product, a crude propylene oxide product is fed to a first azeotropic rectification column to conduct an azeotropic rectification, wherein the feeding location is below a location that is away from the outlet of the column top by 1/4 of the distance from the outlet of the column bottom to the outlet of the column top and above a location that is away from the outlet of the column bottom by 1/4 of the distance from the outlet of the column bottom to the outlet of the column top, and is between the outlet of the column bottom and the outlet of the column top. In another embodiment, the feeding location is below a location that is away from the outlet of the column top by 1/3 of the distance from the outlet of the column bottom to the outlet of the column top and above a location that is away from the outlet of the column bottom by 1/3 of the distance from the outlet of the column bottom to the outlet of the column top, and is between the outlet of the column bottom and the outlet of the column top. In another embodiment, the feeding location is below a location that is away from the outlet of the column top by 2/5 of the distance from the outlet of the column bottom to the outlet of the column top and above a location that is away from the outlet of the column bottom by 2/5 of the distance from the outlet of the column bottom to the outlet of the column top, and is between the outlet of the column bottom and the outlet of the column top. In another embodiment, the feeding location is below a location that is away from the outlet of the column top by 5/12 of the distance from the outlet of the column bottom to the outlet of the column top and above a location that is away from the outlet of the column bottom by 5/12 of the distance from the outlet of the column bottom to the outlet of the column top, and is between the outlet of the column bottom and the outlet of the column top. In another embodiment, the feeding location is at a location that is away from the outlet of the column top by about 1/2 of the distance from the outlet of the column bottom to the outlet of the column top.

According to the present process for refining a crude propylene oxide product, an azeotrope discharged from the column top of said first azeotropic rectification column in the step (1) is fed to a second azeotropic rectification column to conduct an azeotropic rectification, wherein the feeding location is below a location that is away from the outlet of the column top by 1/4 of the distance from the outlet of the column bottom to the outlet of the column top and above a location that is away from the outlet of the column bottom by 1/4 of the distance from the outlet of the column bottom to the outlet of the column top, and is between the outlet of the column bottom and the outlet of the column top. In another embodiment, the feeding location is below a location that is away from the outlet of the column top by 1/3 of the distance from the outlet of the column bottom to the outlet of the column top and above a location that is away from the outlet of the column bottom by 1/3 of the distance from the outlet of the column bottom to the outlet of the column top, and is between the outlet of the column bottom and the outlet of the column top. In another embodiment, the feeding location is below a location that is away from the outlet of the column top by 2/5 of the distance from the outlet of the column bottom to the outlet of the column top and above a location that is away from the outlet of the column bottom by 2/5 of the distance from the outlet of the column bottom to the outlet of the column top, and is between the outlet of the column bottom and the outlet of the column top. In another embodiment, the feeding location is below a location that is away from the outlet of the column top by 5/12 of the distance from the outlet of the column bottom to the outlet of the column top and above a location that is away from the outlet of the column bottom by 5/12 of the distance from the outlet of the column bottom to the outlet of the column top, and is between the outlet of the column bottom and the outlet of the column top. In another embodiment, the feeding location is at a location that is away from the outlet of the column top by about 1/2 of the distance from the outlet of the column bottom to the outlet of the column top.

According to the present process for refining a crude propylene oxide product, a residual part of methanol discharged from the column bottom of said first azeotropic rectification column in the step (1) has a purity of 50-99wt%; a residual part of propylene oxide discharged from the column bottom of said second azeotropic rectification column in the step (2) has a purity of more than or equal to 95wt%, preferably 99wt% or more.

In another aspect of the present invention, the present invention provides a process for preparing propylene oxide, wherein said process for preparing propylene oxide comprises: propylene and $H_2O_2$ are contacted and reacted under a propylene epoxidation reaction condition in the presence of a solid catalyst and methanol and optionally the residual propylene after the reaction is removed to produce a crude propylene oxide product, and the resulting crude propylene oxide product is refined, wherein the resulting crude propylene oxide product is refined with the process for refining a crude propylene oxide product as provided in the present invention. Except that the crude propylene oxide product is refined with the refining process according to the present invention, the process of propylene and $H_2O_2$ being contacted and reacted under a propylene epoxidation reaction condition in the presence of a solid catalyst and methanol to produce the crude propylene oxide product and the specific operation condition thereof can be those well known to a person skilled in the art.

In order to provide the economy for the process for preparing propylene oxide, the residual propylene is removed after the reaction, and then the crude propylene oxide product is refined. The removal of propylene can be conducted in a depropenizing column that is conventionally used in the art.

In addition, said epoxidation reaction can be conducted in a continuous reactor or in a batch reactor. Said reactor can be a fixed bed reactor, a moving bed reactor (or a fluidized bed reactor), and a slurry bed reactor or reaction vessel, which are conventionally used in the art.

Surprisingly, the inventors of the present invention have found that if the propylene epoxidation reaction is only conducted in a moving bed reactor or in a fixed bed reactor, the conversion of $H_2O_2$ is relatively low, and if the propylene epoxidation reaction is only conducted in a slurry bed reactor, the selectivity for propylene oxide is relatively low. The inventors of the present invention have also found that if the propylene epoxidation reaction is conducted in two reactors in series, and if in the flowing direction of the reaction stream, the first reactor is a fixed bed reactor or a moving bed reactor and the second reactor is a slurry bed reactor, the problems of the low conversion of $H_2O_2$ and the low selectivity for propylene oxide can be overcome, and the conversion of $H_2O_2$ and the selectivity for propylene oxide can be improved remarkably.

Therefore, it is preferable that the contact and reaction of propylene and $H_2O_2$ in the presence of a solid catalyst and methanol comprises a mixed stream containing methanol, propylene and $H_2O_2$ is charged successively into a first reactor and a second reactor. It is preferable in the present invention that said first reactor is a fixed bed reactor or a moving bed reactor, and said second reactor is a slurry bed reactor.

According to the present invention, the fixed bed reactor is a reactor widely used in the industry. By the term "fixed bed reactor" is meant a device where the reaction occurs while the fluid flows through the bed formed by the immobile solid materials. In a fixed bed reactor, the catalyst is fixed in the reactor and not mixed with the reactants, and therefore the side reactions are not apt to occur upon using the fixed bed reactor. The selectivity for the target alkylene oxide (e.g. propylene oxide) can be ensured; but the existing problem is the relatively low conversion of $H_2O_2$.

According to the present invention, the moving bed reactor is a reactor that can accomplish the continuous feeding and discharging in a gas-solid phase reaction process or in a liquid-solid phase reaction process. In the moving bed reactor, the back mixing of the reaction materials is little, and therefore the side reactions are not apt to occur upon using a moving bed reactor. The selectivity for the target alkylene oxide (e.g. propylene oxide) can be ensured; but the existing problem is the relatively low conversion of $H_2O_2$.

According to the present invention, the slurry bed reactor is a reactor in which small solid catalyst particles are suspended in the liquid medium. The slurry reactor has a large back mixing of the reaction materials. After the reaction, the catalyst should generally be separated from the reaction materials to be reused. In the slurry bed reactor, the catalyst and the reaction materials are mixed together, and therefore the conversion of $H_2O_2$ is relatively high. However due to the back mixing, the side reactions are apt to occur, which results in a low yield of the target alkylene oxide (e.g. propylene oxide).

According to an embodiment of the present process for preparing propylene oxide, said first reactor is a fixed bed reactor, and said second reactor is a slurry bed reactor. In this embodiment, in said fixed bed reactor, the conditions of said propylene epoxidation reaction can include: the temperature is 30-90°C, preferably 40-80°C, the pressure is 0.5-4.5MPa, preferably 0.6-3MPa, the molar ratio of methanol, propylene and $H_2O_2$ in the feeding is 4-15:0.5-5:1, preferably 5-12:1-3:1, more preferably 5-10:1.5-2.5:1, the liquid volume hourly space velocity of said mixed stream containing methanol, propylene and $H_2O_2$ is 1-15h$^{-1}$, preferably 2-10h$^{-1}$; in said slurry bed reactor, the conditions of said propylene epoxidation reaction can include: the temperature is 30-90°C, preferably 40-80°C, the pressure is 0.5-4.5MPa, preferably 0.6-3MPa, the reaction time is 0.2-10 hrs, preferably 0.4-4 hrs, and with respect to 100 parts by weight of the stream coming from said fixed bed reactor and coming into said slurry bed reactor, the used amount of said solid catalyst is 3-10 parts by weight, preferably 4-9 parts by weight.

According to another embodiment of the present process for preparing propylene oxide, said first reactor is a moving bed reactor, and said second reactor is a slurry bed reactor. In this embodiment, in said moving bed reactor and in said slurry bed reactor, the conditions of said propylene epoxidation reaction can respectively include: the temperature is 30-90°C, preferably 40-80°C, the pressure is 0.5-4.5MPa, preferably 0.6-3MPa, the reaction time is 0.2-10 hrs, preferably 0.4-4 hrs. Moreover, in said moving bed reactor, the molar ratio of methanol, propylene and $H_2O_2$ in the feeding is 4-15:0.5-5:1, preferably 5-12:1-3:1, more preferably 5-10:1.5-2.5:1; in said moving bed reactor, with respect to 100 parts by weight of the mixed stream coming into said moving bed reactor and containing methanol, propylene and $H_2O_2$, the used amount of said solid catalyst is 3-10 parts by weight, preferably 4-9 parts by weight; in said slurry bed reactor, with respect to 100 parts by weight of the stream coming from moving bed reactor and coming into said slurry bed reactor, the used amount of said solid catalyst is 3-10 parts by weight, preferably 4-9 parts by weight.

According to another embodiment of the present process for preparing propylene oxide, wherein said first reactor is a fixed bed reactor or a moving bed reactor, and said second reactor is a slurry bed reactor, in order to further improve the selectivity for propylene oxide and reduce the production of by-products, the process for preparing propylene oxide according to the present invention further comprises: before the resulting reaction product obtained from the first reactor comes into said second reactor, the resulting reaction product obtained from the first reactor is subjected to a separation to obtain a first stream free of $H_2O_2$ and a second stream containing the unreacted $H_2O_2$, wherein said first stream free of $H_2O_2$ contains propylene and propylene oxide, and said second stream containing the unreacted $H_2O_2$ further contains the solvent and water; propylene is added to said second stream, and said second stream to which propylene has been added is charged into said second reactor. In an embodiment, the weight ratio of propylene added to said second stream to $H_2O_2$ in said second stream is 1-2:1.

According to another embodiment of the present process for preparing propylene oxide, wherein said first reactor is a fixed bed reactor or a moving bed reactor, and said second reactor is a slurry bed reactor, in order to further improve the selectivity for propylene oxide and reduce the production of by-products, the process for preparing propylene oxide according to the present invention further comprises: before the resulting reaction product obtained from the first reactor comes into said second reactor, propylene is supplemented to the reaction product obtained in said first reactor, the weight ratio of the supplemented propylene to $H_2O_2$ in the reaction product obtained from said first reactor is 1-2:1.

According to another embodiment of the present process for preparing propylene oxide, in order to obtain a suitable conversion of $H_2O_2$ and a suitable selectivity for propylene oxide, in said first reactor, said propylene epoxidation reaction is conducted so that the conversion rate of $H_2O_2$ reaches 50%-95%; in said second reactor, said propylene epoxidation reaction is conducted so that the total conversion rate of $H_2O_2$ reaches 98% or more, with a proviso that said process for preparing propylene oxide has a selectivity for propylene oxide of 90% or more.

In addition, according to the present invention, said $H_2O_2$ can be added in a form of an aqueous solution, in which the concentration of said $H_2O_2$ can be 10-70wt%, preferably 20-50wt%. In the case of said $H_2O_2$ is added in a form of an aqueous solution, the weight or the molar amount of said $H_2O_2$ is calculated as $H_2O_2$.

According to the present invention, the type of said solid catalyst (including the solid catalyst in the first reactor and the solid catalyst in the second reactor in the preferable process) is not particularly limited, and can be suitably selected from various catalysts conventionally used in the olefin epoxidation process. According to the preferable process for preparing propylene oxide, the solid catalyst in said first reactor and the solid catalyst in said second reactor can be identical or different.

Preferably, said solid catalyst is a titanium silicate catalyst. Specifically, said titanium silicate, for example, can be at least one of a MFI-type titanium silicate, a MEL-type titanium silicate, a BETA-type titanium silicate and a ZSM-12-type titanium silicate. In general, said titanium silicate has a structural formula of $xTiO_2 \cdot SiO_2$, wherein x can be 0.0001-0.04, preferably 0.01-0.03. According to the present invention, said titanium silicate can be commercially available or can be prepared according to the known method. The method of preparing said titanium silicate is well known to a person skilled in the art. For example, said titanium silicate can be prepared by the method as disclosed in the Chinese patent application CN101279959A. In order to further improve the conversion of $H_2O_2$ and the selectivity for propylene oxide in the propylene epoxidation, it is preferable that said catalyst is a molecular sieve of which the crystalline grain is hollow in structure, wherein the radial length of the hollow part of the hollow crystalline grain is 5 to 300nm; under the conditions of 25°C, $P/P_0$ being equal to 0.10 and adsorption time being one hour, the benzene adsorptive capacity of said titanium silicate is at least 70mg/g; and a hysteresis loop exists between the adsorption isotherm and the desorption isotherm of cryogenic nitrogen adsorption of said titanium silicate. According to the present invention, if said preferable catalyst is a molecular sieve of which the crystalline grain is hollow in structure, the reaction materials can easily come into the hollow part of said catalyst to contact and react with the active components of said titanium silicate, which results in the further improvement in the activity of the catalyst; meanwhile propylene oxide can also easily fall off the active sites of said titanium silicate and diffuse into the hollow part of said titanium silicate, which results in the reduced residence time of propylene oxide on the active sites of said titanium silicate, and therefore the reduced probability of the side reaction of propylene oxide and the improved selectivity for propylene oxide. More information about said hollow titanium silicate is disclosed in the Chinese patent application CN101274922A.

According to a preferable embodiment of the present invention, said mixed stream containing methanol, propylene and $H_2O_2$ further contains a surfactant. Based on the total weight of said mixed stream, the content of said surfactant is 0.1-1wt%. Said surfactant can be an oil-soluble surfactant and/or a water-soluble surfactant, for example, can be Span 80 and/or Tween 80. The addition of surfactant into the reaction system can remarkably improve the selectivity for propylene oxide and the use life of the catalyst and reduce the production of by-products.

According to the present invention, the conversion of $H_2O_2$, the total conversion of $H_2O_2$, and the selectivity for the target alkylene oxide (e.g. propylene oxide) are calculated as follows:

$$\text{Conversion of } H_2O_2 \text{ in the step (1)} = \frac{\text{The mole amount of } H_2O_2 \text{ converted in the step (1)}}{\text{The mole amount of } H_2O_2 \text{ fed in the step (1)}} \times 100\% \quad (I)$$

$$\text{Total conversion of } H_2O_2 = \frac{\text{The total mole amount of the converted } H_2O_2}{\text{The total mole amount of the fed } H_2O_2} \times 100\% \quad (II)$$

$$\text{Selectivity for the target alkylene oxide} = \frac{\text{The mole amount of the produced target alkylene oxide}}{\text{The total mole amount of the produced alkylene oxide}} \times 100\% \quad (III)$$

wherein the mole amount of $H_2O_2$, the mole amount of the target alkylene oxide (e.g. propylene oxide), and the total mole amount of the produced alkylene oxide can be measured by the methods well known to a person skilled in the art. For example, the mole amount of $H_2O_2$ can be measured by the iodometry; and the mole amount of the target alkylene oxide (e.g. propylene oxide) and the total mole amount of the produced alkylene oxide can be measured by the chromatography internal standard method.

Hereinafter, the specific examples are provided to further illustrate the present invention. However, the scope of the present invention is not limited by these examples.

The shaped titanium silicate catalyst, as used in the following examples, was prepared as follows:

100g of titanium silicate powder (purchased from Hunan Jianchang Petrochemical Co., Ltd., HTS), 1g of MgO and 40g of tetramethoxysilane were mixed evenly. Then to this mixture were added 20g of silica sol (having a $SiO_2$ content of 30wt%), 2g of polyvinyl alcohol (commercially available from Sanming Dinghui Chemical Trading Co., Ltd., Model 2099), 1g of sesbania powder (purchased from Zhuwa sesbania gum plant, Dongming County, Heze City, Shandong, China) and 20mL of water. The resulting mixture was mixed evenly, extruded to shape, and cut into the shaped particles in a size of 2mm x 2mm. The shaped particles were then dried at 70°C for 4 hrs to produce the shaped bodies A.

100g of the shaped bodies A were placed into a three-necked flask, to which was added 200mL of 20wt% NaOH aqueous solution. The resulting mixture was heated to 90°C while stirring and kept at the same temperature for 6 hrs. Then the resulting solid was washed with deionized water until the washing liquor did not contain sodium cation, and then dried at 120 °C for 3 hrs and calcined at 550 °C for 3 hrs to produce the calcined bodies B.

100g of the calcined bodies B were placed into a three-necked flask, to which was added 200mL of 20wt% NaOH aqueous solution and 10mL of 27.5wt% $H_2O_2$ aqueous solution. The resulting mixture was heated to reflux for 2hrs. Then the resulting solid was washed with deionized water until the washing liquor did not contain sodium cation, and finally dried at 120 °C for 3 hrs and calcined at 550 °C for 5 mins to produce the titanium silicate catalyst.

Example 1

This example illustrates the process for refining a crude propylene oxide product according to the present invention.

(1) The preparation of the crude propylene oxide product

The above titanium silicate catalyst was loaded in a fixed bed reactor, wherein the loading amount of said titanium silicate catalyst was 15mL, and the ceramic ring packing was loaded above and below said catalyst.

Methanol, propylene and $H_2O_2$ having a molar ratio of 7:2:1 were fed to the above fixed bed reactor containing the molecular sieve catalyst in a liquid volume hourly space velocity of $1.2h^{-1}$ to contact an epoxidation reaction, wherein the reaction temperature was 40°C, and the pressure was 2MPa. The residual propylene after the reaction was removed until the content of propylene was less than 1wt% to produce a crude propylene oxide product. In said crude propylene oxide product, the weight concentration of propylene oxide was 13.77wt%, and the weight concentration of methanol was 58.39wt%.

(2) The refining of the crude propylene oxide product

As shown in Fig. 1 (wherein the arrow directions was the flowing direction of the stream), the crude propylene oxide product (stream 1) as obtained in the step (1) was fed to the first azeotropic rectification column T1 to conduct a rectification separation. The operation conditions in said first azeotropic rectification column T1 comprised: the pressure at the column top was 0.29MPa, the temperature at the column top was 65.5°C, the temperature at the column bottom was 103.6°C, the refluxing ratio was 3.2, the column plate number of said first azeotropic rectification column was 36, and the feeding location was at the 18th column plate (counted from the column bottom). 99.78% of propylene oxide coming into said first azeotropic rectification column and 2.09% of methanol coming into said first azeotropic rectification column left from the column top of said first azeotropic rectification column in an azeotrope form, wherein the azeotrope discharged from the column top of said first azeotropic rectification column T1 was composed of 95.73wt% propylene oxide and 4.24wt% methanol. In the mixture (stream 3) discharged from the column bottom of said first azeotropic rectification column T1, the content of propylene oxide was 0.07wt%, the content of methanol was 67.64wt%. The mixture (stream 2) discharged from the column top of said first azeotropic rectification column T1 was fed to said second azeotropic rectification column T2 to conduct a rectification separation. The operation conditions in said second azeotropic rectification column T2 comprised: the pressure at the column top was 1.02MPa, the temperature at the column top was 116°C, the temperature at the column bottom was 119.5°C, the refluxing ratio was 1.3, the column plate number of said second azeotropic rectification column was 18, and the feeding location was at the 9th column plate (counted from the column bottom). 51.25% of propylene oxide coming into said second azeotropic rectification column and 95.66% of methanol coming into said second azeotropic rectification column left from the column top of said second azeotropic rectification column in an azeotrope form, wherein the azeotrope discharged from the column top of said second azeotropic rectification column T2 was composed of 92.31wt% propylene oxide and 7.64wt% methanol. The mixture (stream 5) discharged from the column top of said second azeotropic rectification column T2 was returned to said first azeotropic rectification column T1 to repeat the rectification separation. A propylene oxide product (stream 4) discharged from the column bottom of said second azeotropic rectification column T2 had a purity of 99.60%, wherein the content of methanol was 0.39wt%, and the content of water was less than 0.01%.

Example 2

This example illustrates the process for refining a crude propylene oxide product according to the present invention.
According to the process of Example 1, a crude propylene oxide product was prepared, and the crude propylene oxide product was rectified, except that the operation conditions in said first azeotropic rectification column T1 comprised: the pressure at the column top was 0.25MPa, the temperature at the column top was 62.2°C, the temperature at the column bottom was 104.9°C, the refluxing ratio was 3.2, the column plate number of said first azeotropic rectification column was 36, and the feeding location was at the 18th column plate (counted from the column bottom). The azeotrope from the column top of said first azeotropic rectification column T1 was composed of 95.6wt% of propylene oxide, and 4.4wt% of methanol. In the mixture (stream 3) discharged from the column bottom of said first azeotropic rectification column T1, the content of propylene oxide was 0.4wt%, the content of methanol was 67.3wt%. The mixture (stream 2) discharged from the column top of said first azeotropic rectification column T1 was fed to said second azeotropic rectification column T2 to conduct a rectification separation. The operation conditions in said second azeotropic rectification column T2 comprised: the pressure at the column top was 0.95MPa, the temperature at the column top was 114°C, the temperature at the column bottom was 116.2°C, the refluxing ratio was 1.3, the column plate number of said second azeotropic rectification column was 20, and the feeding location was at the 10th column plate (counted from the column bottom). The azeotrope discharged from the column top of said second azeotropic rectification column T2 was composed of 94.2wt% of propylene oxide, and 5.8wt% of methanol. The mixture (stream 5) discharged from the column top of said second azeotropic rectification column T2 was returned to said first azeotropic rectification column T1 to repeat the rectification separation. A propylene oxide product (stream 4) discharged from the column bottom of said second azeotropic rectification column T2 had a purity of 99.3%, wherein the content of methanol was 0.7wt%.

Example 3

This example illustrates the process for refining a crude propylene oxide product according to the present invention.
According to the process of Example 1, a crude propylene oxide product was prepared, and the crude propylene oxide product was rectified, except that the operation conditions in said first azeotropic rectification column T1 comprised: the pressure at the column top was 0.4MPa, the temperature at the column top was 78.7°C, the temperature at the column bottom was 117.4°C, the refluxing ratio was 3.2, the column plate number of said first azeotropic rectification column was 40, and the feeding location was at the 20th column plate (counted from the column bottom). The azeotrope from the column top of said first azeotropic rectification column T1 was composed of 93.8wt% of propylene oxide, and 6.2wt% of methanol. In the mixture (stream 3) discharged from the column bottom of said first azeotropic rectification column T1, the content of propylene oxide was 0.7wt%, and the content of methanol was 67wt%. The mixture (stream 2) discharged from the column top of said first azeotropic rectification column T1 was fed to said second azeotropic rectification column T2 to conduct a rectification separation. The operation conditions in said second azeotropic rectification column T2 comprised: the pressure at the column top was 1.02MPa, the temperature at the column top was 117.1°C, the temperature at the column bottom was 119.0°C, the refluxing ratio was 1.3, the column plate number of said second azeotropic rectification was 18, and the feeding location was at the 9th column plate (counted from the column bottom). The azeotrope discharged from the column top of said second azeotropic rectification column T2 was composed of 92.1wt% of propylene oxide and 7.9wt% of methanol. The mixture (stream 5) discharged from the column top of said second azeotropic rectification column T2 was returned to said first azeotropic rectification column T1 to repeat the rectification separation. A propylene oxide product (stream 4) discharged from the column bottom of said second azeotropic rectification column T2 had a purity of 98.5%, wherein the content of methanol was 1.5wt%.

Example 4

This example illustrates the process for refining a crude propylene oxide product according to the present invention.
According to the process of Example 1, a crude propylene oxide product was prepared, and the crude propylene oxide product was rectified, except that the operation conditions in said first azeotropic rectification column T1 comprised: the pressure at the column top was 0.5MPa, the temperature at the column top was 87.2°C, the temperature at the column bottom was 124.0°C, the refluxing ratio was 3.2, the column plate number of said first azeotropic rectification column was 40, and the feeding location was at the 20th column plate (counted from the column bottom). The azeotrope from the column top of said first azeotropic rectification column T1 was composed of 92.8wt% of propylene oxide, and 7.2wt% of methanol. In the mixture (stream 3) discharged from the column bottom of said first azeotropic rectification column T1, the content of propylene oxide was 0.9wt%, the content of methanol was 66.8wt%. The mixture (stream 2) discharged from the column top of said first azeotropic rectification column T1 was fed to said second azeotropic rectification column T2 to conduct a rectification separation. The operation conditions in said second azeotropic rectification column T2 comprised: the pressure at the column top was 1MPa, the temperature at the column top was 116.2°C, the temperature at the column bottom was 117.9°C, the refluxing ratio was 1.3, the column plate number of said second azeotropic rectification column was 18, and the feeding location was at the 9th column plate (counted from the column bottom). The azeotrope discharged from the column top of said second azeotropic rectification column T2 was composed of 91.3wt% of propylene oxide, and 8.7wt% of methanol. The mixture (stream 5) discharged from the column top of said second azeotropic rectification column T2 was returned to said first azeotropic rectification column T1 to repeat the rectification separation. A propylene oxide product (stream 4) discharged from the column bottom of said second azeotropic rectification column T2 had a purity of 97.4%, wherein the content of methanol was 2.6wt%.

Example 5

This example illustrates the process for refining a crude propylene oxide product according to the present invention.

According to the process of Example 1, a crude propylene oxide product was prepared, and the crude propylene oxide product was rectified, except that the operation conditions in said first azeotropic rectification column T1 comprised: the pressure at the column top was 0.8MPa, the temperature at the column top was 106.4°C, the temperature at the column bottom was 139.5°C, the refluxing ratio was 4, the column plate number of said first azeotropic rectification column was 32, and the feeding location was at the 16th column plate (counted from the column bottom). The azeotrope from the column top of said first azeotropic rectification column T1 was composed of 91.8wt% of propylene oxide, and 8.2wt% of methanol. In the mixture (stream 3) discharged from the column bottom of said first azeotropic rectification column T1, the content of propylene oxide was 1.2wt%, the content of methanol was 66.5wt%. The mixture (stream 2) discharged from the column top of said first azeotropic rectification column T1 was fed to said second azeotropic rectification column T2 to conduct a rectification separation. The operation conditions in said second azeotropic rectification column T2 comprised: the pressure at the column top was 0.9MPa, the temperature at the column top was 111.5°C, the temperature at the column bottom was 113.2°C, the refluxing ratio was 2, the column plate number of said second azeotropic rectification column was 25, and the feeding location was at the 12th column plate (counted from the column bottom). The azeotrope discharged from the column top of said second azeotropic rectification column T2 was composed of 91.1wt% of propylene oxide, and 8.9wt% of methanol. The mixture (stream 5) discharged from the column top of said second azeotropic rectification column T2 was returned to said first azeotropic rectification column T1 to repeat the rectification separation. A propylene oxide product discharged from the side outlet of said second azeotropic rectification column T2 had a purity of 96.5%, wherein the content of methanol was 3.5wt%.

Example 6

This example illustrates the process for preparing propylene oxide according to the present invention.

(1) The preparation of the crude propylene oxide product

The same titanium silicate catalyst as that used in Example 1 was loaded in a fixed bed reactor (purchased from Penglai Luhao Chemical Machinery Co., Ltd.). The loading amount of said titanium silicate catalyst was 15mL. A mixed stream containing propylene, methanol and $H_2O_2$ was fed to the fixed bed reactor in a liquid volume hourly space velocity of $7h^{-1}$, wherein the molar ratio of methanol, propylene and $H_2O_2$ was 8:4:1. In said fixed bed reactor, the temperature was 70°C, and the pressure was 2MPa. The reaction product obtained in the fixed bed reactor was introduced to a separation column to conduct a separation to obtain a first stream containing propylene and propylene oxide but free of $H_2O_2$, and a second stream containing $H_2O_2$, methanol and water. The content of $H_2O_2$ in said second stream was measured by iodometry. Then, with respect to 100 parts by weight of $H_2O_2$ in said second stream, 150 parts by weight of propylene was added to the second stream.

The second stream to which propylene had been added and the titanium silicate powder (purchased from Hunan Jianchang Petrochemical Co., Ltd., HTS) in a weight ratio of 100:5 were introduced to a slurry bed reactor (purchased from purchased from Tianjin Aozhan Technology Co,. Ltd.). In the slurry bed reactor, the temperature was 70°C, and the pressure was 2MPa. The second stream, to which propylene had been added, after the residence of 1hr in said slurry bed reactor, was subjected to a separation together with said first stream to separate propylene off to produce a crude propylene oxide product (having a propylene content of less than 1wt%). The crude propylene oxide product was analyzed, and the conversion of $H_2O_2$ and the selectivity for propylene oxide were calculated. The results were shown in Table 1.

(2) The refining of the crude propylene oxide product

As shown in Fig. 1 (wherein the arrow directions was the flowing direction of the stream), the crude propylene oxide product (stream 1) as obtained in the above step (1) was fed to the first azeotropic rectification column T1 to conduct a rectification separation. The operation conditions in said first azeotropic rectification column T1 comprised: the pressure at the column top was 0.29MPa, the temperature at the column top was 65.5°C, the temperature at the column bottom was 103.6°C, the refluxing ratio was 3.2, the column plate number of said first azeotropic rectification column was 36, and the feeding location was at the 18th column plate (counted from the column bottom). The azeotrope from the column top of said first azeotropic rectification column T1 was composed of 95.73wt% of propylene oxide, and 4.27wt% of methanol. In the mixture (stream 3) discharged from the column bottom of said first azeotropic rectification column T1, the content of propylene oxide was 0.07wt%, the content of methanol was 67.64wt%. The mixture (stream 2) discharged from the column top of said first azeotropic rectification column T1 was fed to said second azeotropic rectification column T2 to conduct a rectification separation. The operation conditions in said second azeotropic rectification column T2 comprised: the pressure at the column top was 1.02MPa, the temperature at the column top was 116°C, the temperature at the column bottom was 119.5°C, the refluxing ratio was 1.3, the column plate number of said second azeotropic rectification column was 18, and the feeding location was at the 9th column plate (counted from the column bottom). The azeotrope discharged from the column top of said second azeotropic rectification column T2 was composed of 92.3wt% of propylene oxide, and 7.64wt% of methanol. The mixture (stream 5) discharged from the column top of said second azeotropic rectification column T2 was returned to said first azeotropic rectification column T1 to repeat the rectification separation. A propylene oxide product (stream 4) discharged from the column bottom of said second azeotropic rectification column T2 had a purity of 99.7%, wherein the content of methanol was 0.3wt%.

Example 7

This example illustrates the process for preparing propylene oxide according to the present invention.
According to the process of Example 6, a crude propylene oxide product was prepared, and the crude propylene oxide product was refined, except that in the refining process of the crude propylene oxide product, the operation conditions in said first azeotropic rectification column T1 comprised: the pressure at the column top was 0.25MPa, the temperature at the column top was 62.2°C, the temperature at the column bottom was 104.9°C, the refluxing ratio was 3.2, the column plate number of said first azeotropic rectification column was 36, and the feeding location was at the 18th column plate (counted from the column bottom). The azeotrope from the column top of said first azeotropic rectification column T1 was composed of 95.6wt% of propylene oxide, and 4.4wt% of methanol. In the mixture (stream 3) discharged from the column bottom of said first azeotropic rectification column T1, the content of propylene oxide was 0.4wt%, the content of methanol was 67.3wt%. The mixture (stream 2) discharged from the column top of said first azeotropic rectification column T1 was fed to said second azeotropic rectification column T2 to conduct a rectification separation. The operation conditions in said second azeotropic rectification column T2 comprised: the pressure at the column top was 0.95MPa, the temperature at the column top was 114°C, the temperature at the column bottom was 116.2°C, the refluxing ratio was 1.3, the column plate number of said second azeotropic rectification column was 20, and the feeding location was at the 10th column plate (counted from the column bottom). The azeotrope discharged from the column top of said second azeotropic rectification column T2 was composed of 94.2wt% of propylene oxide, and 5.8wt% of methanol. The mixture (stream 5) discharged from the column top of said second azeotropic rectification column T2 was returned to said first azeotropic rectification column T1 to repeat the rectification separation. A propylene oxide product (stream 4) discharged from the column bottom of said second azeotropic rectification column T2 had a purity of 99.3%, wherein the content of methanol was 0.7wt%.

Example 8

This example illustrates the process for preparing propylene oxide according to the present invention.
According to the process of Example 6, a crude propylene oxide product was prepared, and the crude propylene oxide product was refined, except that in the refining process of the crude propylene oxide product, the operation conditions in said first azeotropic rectification column T1 comprised: the pressure at the column top was 0.4MPa, the temperature at the column top was 78.7°C, the temperature at the column bottom was 117.4°C, the refluxing ratio was 3.2, the column plate number of said first azeotropic rectification column was 40, and the feeding location was at the 20th column plate (counted from the column bottom). The azeotrope from the column top of said first azeotropic rectification column T1 was composed of 93.8wt% of propylene oxide, and 6.2wt% of methanol. In the mixture (stream 3) discharged from the column bottom of said first azeotropic rectification column T1, the content of propylene oxide was 0.7wt%, the content of methanol was 67wt%. The mixture (stream 2) discharged from the column top of said first azeotropic rectification column T1 was fed to said second azeotropic rectification column T2 to conduct a rectification separation. The operation conditions in said second azeotropic rectification column T2 comprised: the pressure at the column top was 1.02MPa, the temperature at the column top was 117.1°C, the temperature at the column bottom was 119.0°C, the refluxing ratio was 1.3, the column plate number of said second azeotropic rectification was 18, and the feeding location was at the 9th column plate (counted from the column bottom). The azeotrope discharged from the column top of said second azeotropic rectification column T2 was composed of 92.1wt% of propylene oxide, and 7.9wt% of methanol. The mixture (stream 5) discharged from the column top of said second azeotropic rectification column T2 was returned to said first azeotropic rectification column T1 to repeat the rectification separation. A propylene oxide product (stream 4) discharged from the column bottom of said second azeotropic rectification column T2 had a purity of 98.5%, wherein the content of methanol was 1.5wt%.

Example 9

This example illustrates the process for preparing propylene oxide according to the present invention.
According to the process of Example 6, a crude propylene oxide product was prepared, and the crude propylene oxide product was refined, except that in the refining process of the crude propylene oxide product, the operation conditions in said first azeotropic rectification column T1 comprised: the pressure at the column top was 0.5MPa, the temperature at the column top was 87.2°C, the temperature at the column bottom was 124.0°C, the refluxing ratio was 3.2, the column plate number of said first azeotropic rectification column was 40, and the feeding location was at the 20th column plate (counted from the column bottom). The azeotrope from the column top of said first azeotropic rectification column T1 was composed of 92.8wt% of propylene oxide, and 7.2wt% of methanol. In the mixture (stream 3) discharged from the column bottom of said first azeotropic rectification column T1, the content of propylene oxide was 0.9wt%, the content of methanol was 66.8wt%. The mixture (stream 2) discharged from the column top of said first azeotropic rectification column T1 was fed to said second azeotropic rectification column T2 to conduct a rectification separation. The operation conditions in said second azeotropic rectification column T2 comprised: the pressure at the column top was 1MPa, the temperature at the column top was 116.2°C, the temperature at the column bottom was 117.9°C, the refluxing ratio was 1.3, the column plate number of said second azeotropic rectification column was 18, and the feeding location was at the 9th column plate (counted from the column bottom). The azeotrope discharged from the column top of said second azeotropic rectification column T2 was composed of 91.3wt% of propylene oxide, and 8.7wt% of methanol. The mixture (stream 5) discharged from the column top of said second azeotropic rectification column T2 was returned to said first azeotropic rectification column T1 to repeat the rectification separation. A propylene oxide product (stream 4) discharged from the column bottom of said second azeotropic rectification column T2 had a purity of 97.4%, wherein the content of methanol was 2.6wt%.

Example 10

This example illustrates the process for preparing propylene oxide according to the present invention.
According to the process of Example 6, a crude propylene oxide product was prepared, and the crude propylene oxide product was refined, except that in the refining process of the crude propylene oxide product, the operation conditions in said first azeotropic rectification column T1 comprised: the pressure at the column top was 0.8MPa, the temperature at the column top was 106.4°C, the temperature at the column bottom was 139.5°C, the refluxing ratio was 4, the column plate number of said first azeotropic rectification column was 32, and the feeding location was at the 16th column plate (counted from the column bottom). The azeotrope from the column top of said first azeotropic rectification column T1 was composed of 91.8wt% of propylene oxide, and 8.2wt% of methanol. In the mixture (stream 3) discharged from the column bottom of said first azeotropic rectification column T1, the content of propylene oxide was 1.2wt%, the content of methanol was 66.5wt%. The mixture (stream 2) discharged from the column top of said first azeotropic rectification column T1 was fed to said second azeotropic rectification column T2 to conduct a rectification separation. The operation conditions in said second azeotropic rectification column T2 comprised: the pressure at the column top was 0.9MPa, the temperature at the column top was 111.5°C, the temperature at the column bottom was 113.2°C, the refluxing ratio was 2, the column plate number of said second azeotropic rectification column was 25, and the feeding location was at the 12th column plate (counted from the column bottom). The azeotrope discharged from the column top of said second azeotropic rectification column T2 was composed of 91.1wt% of propylene oxide, and 8.9wt% of methanol. The mixture (stream 5) discharged from the column top of said second azeotropic rectification column T2 was returned to said first azeotropic rectification column T1 to repeat the rectification separation. A propylene oxide product discharged from the side outlet of said second azeotropic rectification column T2 had a purity of 96.5%, wherein the content of methanol was 3.5wt%.

It was seen from Examples 1-10 that the process for preparing propylene oxide according to the present invention could prepare propylene oxide with high purity.

Example 11

This example illustrates the process for preparing propylene oxide according to the present invention.

According to the process of Example 6, a propylene oxide product was prepared, except that in the preparation process of the crude propylene oxide product, the slurry bed reactor was replaced with a fixed bed reactor. Said fixed bed reactor is loaded with 15mL of the same titanium silicate catalyst as that used in Example 1. In said fixed bed reactor, the the temperature was 70°C, and the pressure was 2MPa, The stream through said fixed bed reactor had a liquid volume hourly space velocity of $6h^{-1}$. The obtained crude propylene oxide product was analyzed, and the conversion of $H_2O_2$ and the selectivity for propylene oxide were calculated. The results were shown in Table 1.

Example 12

This example illustrates the process for preparing propylene oxide according to the present invention.

According to the process of Example 6, a propylene oxide product was prepared, except that the preparation process of the crude propylene oxide product was as follows:

The same titanium silicate catalyst as that used in Example 1 was loaded in a fixed bed reactor (purchased from Penglai Luhao Chemical Machinery Co., Ltd.) with a catalyst loading of 15mL. A mixed stream containing propylene, methanol and $H_2O_2$ was introduced into a fixed bed reactor in a liquid volume hourly space velocity of 10h-\ wherein the molar ratio of methanol, propylene and $H_2O_2$ in said mixed stream was 5:1.5:1. In said fixed bed reactor, the temperature was 40°C, and the pressure was 3MPa. Then the content of the reaction product produced in said fixed bed reactor was measured by iodometry and with respect to 100 parts by weight of $H_2O_2$ in said reaction product, 100 parts by weight of propylene was supplemented to said reaction product.

The reaction product to which propylene had been supplemented and the titanium silicate powder (purchased from Hunan Jianchang Petrochemical Co., Ltd., HTS) in a weight ratio of 100:9 were introduced to the slurry bed reactor (purchased from purchased from Tianjin Aozhan Technology Co,. Ltd.). In said slurry bed reactor, the temperature was 40°C, and the pressure was 3MPa. After the reaction product, to which propylene had been supplemented, resided in said slurry bed reactor for 2hrs, a crude propylene oxide product was obtained. The crude propylene oxide product was analyzed, and the conversion of $H_2O_2$ and the selectivity for propylene oxide were calculated. The results were shown in Table 1.

Example 13

This example illustrates the process for preparing propylene oxide according to the present invention.

According to the process of Example 6, a propylene oxide product was prepared, except that the preparation process of the crude propylene oxide product was as follows:

The same titanium silicate catalyst as that used in Example 1 was loaded in a fixed bed reactor (purchased from Penglai Luhao Chemical Machinery Co., Ltd.) with a catalyst loading of 15mL. A mixed stream containing propylene, methanol and $H_2O_2$ was introduced into a fixed bed reactor in a liquid volume hourly space velocity of $2h^{-1}$, wherein the molar ratio of methanol, propylene and $H_2O_2$ in said mixed stream was 10:2.5:1. In said fixed bed reactor, the temperature was 80°C, and the pressure was 0.6MPa. Then the content of the reaction product produced in said fixed bed reactor was measured by iodometry and with respect to 100 parts by weight of $H_2O_2$ in said reaction product, 100 parts by weight of propylene was supplemented to said reaction product.

The reaction product to which propylene had been supplemented and the titanium silicate powder (purchased from Hunan Jianchang Petrochemical Co., Ltd., HTS) in a weight ratio of 100:4 were introduced to the slurry bed reactor (purchased from purchased from Tianjin Aozhan Technology Co,. Ltd.). In said slurry bed reactor, the temperature was 80°C, and the pressure was 0.6MPa. After the reaction product, to which propylene had been supplemented, resided in said slurry bed reactor for 3hrs, a crude propylene oxide product was obtained. The crude propylene oxide product was analyzed, and the conversion of $H_2O_2$ and the selectivity for propylene oxide were calculated. The results were shown in Table 1.

Example 14

This example illustrates the process for preparing propylene oxide according to the present invention.

According to the process of Example 6, a propylene oxide product was prepared, except that the preparation process of the crude propylene oxide product was as follows:

The titanium silicate powder (purchased from Hunan Jianchang Petrochemical Co., Ltd., HTS) and a mixed stream containing propylene, methanol and $H_2O_2$ in a weight ratio of 100:6 were added to a moving bed reactor (purchased from Chengdu YonTon Machinery Factory), wherein the molar ratio of methanol, propylene and $H_2O_2$ was 8:4:1. In said moving bed reactor, the temperature was 70°C, and the pressure was 2MPa. The reaction product obtained in the moving bed reactor was introduced to a separation column to conduct a separation to obtain a first stream containing propylene and propylene oxide, and a second stream containing $H_2O_2$, methanol and water. The content of $H_2O_2$ in said second stream was measured by iodometry. Then, with respect to 100 parts by weight of $H_2O_2$ in said second stream, 150 parts by weight of propylene was added to the second stream.

The second stream to which propylene had been added and the titanium silicate powder (purchased from Hunan Jianchang Petrochemical Co., Ltd., HTS) in a weight ratio of 100:5 were introduced to the slurry bed reactor (purchased from purchased from Tianjin Aozhan Technology Co,. Ltd.). In said slurry bed reactor, the temperature was 70°C, and the pressure was 2MPa. After the second stream, to which propylene had been added, resided in said slurry bed reactor for 1hr, a crude propylene oxide product was obtained. The crude propylene oxide product was analyzed, and the conversion of $H_2O_2$ and the selectivity for propylene oxide were calculated. The results were shown in Table 1.

Example 15

This example illustrates the process for preparing propylene oxide according to the present invention.

According to the process of Example 6, a propylene oxide product was prepared, except that the preparation process of the crude propylene oxide product was as follows:

The titanium silicate powder (purchased from Hunan Jianchang Petrochemical Co., Ltd., HTS) and a mixed stream containing propylene, methanol and $H_2O_2$ in a weight ratio of 100:9 were added to a moving bed reactor (purchased from Chengdu YonTon Machinery Factory), wherein the molar ratio of methanol, propylene and $H_2O_2$ was 5:1.5:1. In said moving bed reactor, the temperature was 40°C, and the pressure was 3MPa. Then the content of the reaction product produced in said moving bed reactor was measured by iodometry and with respect to 100 parts by weight of $H_2O_2$ in said reaction product, 100 parts by weight of propylene was supplemented to said reaction product.

The reaction product to which propylene had been supplemented and the titanium silicate powder (purchased from Hunan Jianchang Petrochemical Co., Ltd., HTS) in a weight ratio of 100:9 were introduced to the slurry bed reactor (purchased from purchased from Tianjin Aozhan Technology Co,. Ltd.). In said slurry bed reactor, the temperature was 40°C, and the pressure was 3MPa. After the reaction product, to which propylene had been supplemented, resided in said slurry bed reactor for 2hrs, a crude propylene oxide product was obtained. The crude propylene oxide product was analyzed, and the conversion of $H_2O_2$ and the selectivity for propylene oxide were calculated. The results were shown in Table 1.

Example 16

This example illustrates the process for preparing propylene oxide according to the present invention.

According to the process of Example 6, a propylene oxide product was prepared, except that the preparation process of the crude propylene oxide product was as follows:

The titanium silicate powder (purchased from Hunan Jianchang Petrochemical Co., Ltd., HTS) and a mixed stream containing propylene, methanol and $H_2O_2$ in a weight ratio of 100:4 were added to a fixed bed reactor (purchased from Penglai Luhao Chemical Machinery Co., Ltd.), wherein the molar ratio of methanol, propylene and $H_2O_2$ in said mixed stream was 10:2.5:1. In said fixed bed reactor, the temperature was 80°C, and the pressure was 0.6MPa. Then the content of the reaction product produced in said fixed bed reactor was measured by iodometry and with respect to 100 parts by weight of $H_2O_2$ in said reaction product, 200 parts by weight of propylene was supplemented to said reaction product.

The reaction product to which propylene had been supplemented and the titanium silicate powder (purchased from Hunan Jianchang Petrochemical Co., Ltd., HTS) in a weight ratio of 100:4 were introduced to the slurry bed reactor (purchased from purchased from Tianjin Aozhan Technology Co,. Ltd.). In said slurry bed reactor, the temperature was 80°C, and the pressure was 0.6MPa. After the reaction product, to which propylene had been supplemented, resided in said slurry bed reactor for 3hrs, a crude propylene oxide product was obtained. The crude propylene oxide product was analyzed, and the conversion of $H_2O_2$ and the selectivity for propylene oxide were calculated. The results were shown in Table 1.

Table 1

| No. | the total conversion of $H_2O_2$ | the selectivity for propylene oxide |
| --- | --- | --- |
| Example 6 | 99.4% | 98.8% |
| Example 11 | 86.2% | 94.4% |
| Example 12 | 98.1% | 96.0% |
| Example 13 | 97.5% | 95.3% |
| Example 14 | 99.2% | 98.5% |
| Example 15 | 97.9% | 95.8% |
| Example 16 | 96.8% | 95.1% |

It could be seen from Examples that the process of the present invention could produce the propylene oxide product with relative high purity. In particular, it could be seen from the data in the above Table 1 that in the preferable process according to the present invention, the combination of fixed bed reactor and slurry bed reactor or the combination of moving bed reactor and slurry bed reactor could remarkably increase the conversion of $H_2O_2$ and the selectivity for propylene oxide.

**Claims**

1. A process for refining a crude propylene oxide product, wherein said crude propylene oxide product is a mixture containing propylene oxide and methanol, which process is characterized that said process comprises:

(1) in a first azeotropic rectification condition, a crude propylene oxide product is fed to a first azeotropic rectification column to conduct an azeotropic rectification, said first azeotropic rectification condition is such a condition that propylene oxide and methanol in a stream fed to said first azeotropic rectification column are discharged from the column top in an azeotrope form, and a residual part of methanol and propylene oxide are discharged from the column bottom, wherein said residual part of methanol discharged from the column bottom of said first azeotropic rectification column has a purity of 50-99wt%; an effluent from the column bottom of said first azeotropic rectification column has a propylene oxide content of less than 5wt%;

(2) in a second azeotropic rectification condition, an azeotrope discharged from the column top of said first azeotropic rectification column in the step (1) is fed to a second azeotropic rectification column to conduct an azeotropic rectification, said second azeotropic rectification condition is such a condition that methanol and propylene oxide in a mixture fed to said second azeotropic rectification column are discharged from the column top of said second azeotropic rectification column in an azeotrope form, a residual part of propylene oxide and methanol are discharged from the column bottom of said second azeotropic rectification column, wherein said residual part of propylene oxide discharged from the column bottom of said second azeotropic rectification column has a purity of more than or equal to 95wt%; an effluent from the column bottom of said second azeotropic rectification column has a methanol content of less than 5wt%;

(3) optionally, the azeotrope discharged from the column top of said second azeotropic rectification column in the step (2) is partly or totally returned to said first azeotropic rectification column used in the step (1) to conduct an azeotropic rectification together with the crude propylene oxide product,

wherein said first azeotropic rectification condition and said second azeotropic rectification condition include:

the pressure at the column top of said first azeotropic rectification column is 0.2-0.8MPa, and the pressure at the column top of said second azeotropic rectification column is 0.9-1.5MPa,

for said first azeotropic rectification column: the temperature at the column bottom is 100-140°C; the column plate number is 20-45, the refluxing ratio is 2-5:1, the feeding location is below a location that is away from the outlet of the column top by 1/4 of the distance from the outlet of the column bottom to the outlet of the column top and above a location that is away from the outlet of the column bottom by 1/4 of the distance from the outlet of the column bottom to the outlet of the column top, and is between the outlet of the column bottom and the outlet of the column top;

for said second azeotropic rectification column: the temperature at the column bottom is 110-130°C; the column plate number is 15-25, the refluxing ratio is 0.8-3:1, the feeding location is below a location that is away from the outlet of the column top by 1/4 of the distance from the outlet of the column bottom to the outlet of the column top and above a location that is away from the outlet of the column bottom by 1/4 of the distance from the outlet of the column bottom to the outlet of the column top.

2. The process according to claim 1, wherein in said crude propylene oxide product, the content of propylene oxide is 5-99.5wt%, the content of methanol is 0.5-95wt%, based on the total weight of the crude propylene oxide product containing methanol and propylene oxide.

3. The process according to claim 1, wherein an effluent from the column bottom of said first azeotropic rectification column has a propylene oxide content of less than 3wt%, and an effluent from the column bottom of said second azeotropic rectification column has a methanol content of less than 3wt%.

4. The process according to claim 1, wherein said first azeotropic rectification condition and said second azeotropic rectification condition are such conditions that the content of methanol in the azeotrope discharged from the column top of said first azeotropic rectification column is less than the content of methanol in the azeotrope discharged from the column top of said second azeotropic rectification column.

5. The process according to claim 1, wherein in the step (1), in the mixture discharged from the column top of said first azeotropic rectification column, the weight concentration of propylene oxide is 90-98wt%, and the weight concentration of methanol is 2-10wt%; in the step (2), in the mixture discharged from the column top of said second azeotropic rectification column, the weight concentration of propylene oxide is 85wt% to less than 98wt%, and the weight concentration of methanol is more than 2wt% to 15wt%.

6. The process according to claim 1, wherein said first azeotropic rectification condition and said second azeotropic rectification condition further include: the temperature at the column top of said second azeotropic rectification column is more than the temperature at the column top of said first azeotropic rectification column.

7. The process according to claim 6, wherein said first azeotropic rectification condition and said second azeotropic rectification condition include: the temperature at the column top of said first azeotropic rectification column is 60-107°C, and the temperature at the column top of said second azeotropic rectification column is 110-125°C.

8. The process according to any one of Claims 1-7, wherein said crude propylene oxide product is obtained by a method for preparing propylene oxide, which method comprises propylene and $H_2O_2$ are contacted and reacted under a propylene epoxidation reaction condition in the presence of a solid catalyst and methanol and optionally the residual propylene after the reaction is removed to produce said crude propylene oxide product.

9. The process according to Claim 8, wherein the reaction of propylene and $H_2O_2$ in the presence of a solid catalyst and methanol comprises

a mixed stream containing methanol, propylene and $H_2O_2$ is charged successively into a first reactor and a second reactor, wherein said first reactor is a fixed bed reactor or a moving bed reactor, and said second reactor is a slurry bed reactor,

in said first reactor, said propylene epoxidation reaction is conducted so that the conversion rate of $H_2O_2$ reaches 50%-95%; in said second reactor, said propylene epoxidation reaction is conducted so that the total conversion rate of $H_2O_2$ reaches 98% or more, with a proviso that said method for preparing propylene oxide has a selectivity for propylene oxide of 90% or more.

10. The process according to Claim 9, wherein

in said fixed bed reactor, said propylene epoxidation reaction condition includes: the temperature is 30-90°C, the pressure is 0.5-4.5MPa, and the liquid volume hourly space velocity of said mixed stream is 1-15h$^{-1}$;
in said moving bed reactor, said propylene epoxidation reaction condition includes: the temperature is 30-90°C, the pressure is 0.5-4.5MPa, the reaction time is 0.2-10 hrs, with respect to 100 parts by weight of the mixed stream coming into said moving bed reactor and containing methanol, propylene and H$_2$O$_2$, the used amount of said solid catalyst is 3-10 parts by weight;
in said slurry bed reactor, said propylene epoxidation reaction condition includes: the temperature is 30-90°C, the pressure is 0.5-4.5MPa, the reaction time is 0.2-10 hrs, with respect to 100 parts by weight of the stream coming from said fixed bed reactor or moving bed reactor and coming into slurry bed reactor, the used amount of said solid catalyst is 3-10 parts by weight.

11. The process according to Claim 9, wherein said method further comprises before the resulting reaction product obtained from the first reactor comes into said second reactor, the resulting reaction product obtained from the first reactor is subjected to a separation to obtain a first stream free of H$_2$O$_2$ and a second stream containing the unreacted H$_2$O$_2$, wherein said first stream free of H$_2$O$_2$ contains propylene and propylene oxide, and said second stream containing the unreacted H$_2$O$_2$ further contains the solvent and water; propylene is added to said second stream, and said second stream to which propylene has been added is charged into said second reactor, optionally, the weight ratio of propylene added to said second stream to H$_2$O$_2$ in said second stream is 1-3:1.

12. The process according to Claim 9, wherein said method further comprises before the resulting reaction product obtained from the first reactor comes into said second reactor, propylene is supplemented to the reaction product obtained in said first reactor, the weight ratio of the supplemented propylene to H$_2$O$_2$ in the reaction product obtained from said first reactor is 1-2:1.

13. The process according to Claim 9, wherein the molar ratio of methanol, propylene and H$_2$O$_2$ in said mixed stream is 4-15:0.5-5:1.

14. The process according to Claim 9, wherein said solid catalyst is a titanium silicate.

15. The process according to Claim 9, wherein the residual propylene after reaction is removed so that in the resulting crude propylene oxide product, the content of propylene is less than 5wt%, based on the weight of the crude propylene oxide product.

**Patentansprüche**

1. Verfahren zum Raffinieren eines rohen Propylenoxidproduktes, worin das rohe Propylenoxidprodukt eine Mischung ist, umfassend Propylenoxid und Methanol, wobei das Verfahren gekennzeichnet ist, dass das Verfahren umfasst:

(1) bei einer ersten azeotropen Rektifizierbedingung wird ein rohes Propylenoxidprodukt zu einer ersten azeotropen Rektifiziersäule geführt, zur Durchführung einer azeotropen Rektifizierung, wobei die erste azeotrope Rektifizierbedingung eine solche Bedingung ist, dass Propylenoxid und Methanol in einem Strom, der zu der ersten azeotropen Rektifiziersäule geführt wird, aus der Seite der Säule in einer azeotropen Form abgelassen werden, und ein restlicher Teil von Methanol und Propylenoxid vom Säulenboden abgelassen wird, worin der restliche Teil von Methanol, der von dem Säulenboden der ersten azeotropen Rektifiziersäule abgelassen ist, eine Reinheit von 50 bis 99 Gew.% hat; ein Ablauf vom Säulenboden der ersten azeotropen Rektifiziersäule einen Propylenoxidgehalt von weniger als 5 Gew.% hat;
(2) in einer zweiten azeotropen Rektifizierbedingung wird ein Azeotrop, abgelassen von der Säulenspitze der ersten azeotropen Rektifiziersäule im Schritt (1), zu einer zweiten azeotropen Rektifiziersäule geführt, zum Durchführen einer azeotropen Rektifizierung, wobei die zweite azeotrope Rektifizierbedingung eine solche Bedingung ist, dass Methanol und Propylenoxid in einer Mischung, geführt zu der zweiten azeotropen Rektifiziersäule, von der Säulenspitze der zweiten azeotropen Rektifiziersäule in einer azeotropen Form abgelassen werden, ein restlicher Teil von Propylenoxid und Methanol von dem Säulenboden der zweiten azeotropen Rektifiziersäule abgelassen werden, worin der restliche Teil von Propylenoxid, abgelassen von dem Säulenboden der zweiten azeotropen Rektifiziersäule, eine Reinheit von mehr als oder gleich 95 Gew.% hat; ein Ablauf von dem Säulenboden der zweiten azeotropen Rektifiziersäule einen Methanolgehalt von weniger als 5 Gew.% hat;
(3) wahlweise wird das Azeotrop, abgelassen von der Säulenspitze der zweiten azeotropen Rektifiziersäule im Schritt (2) teilweise oder vollständig zu der ersten azeotropen Rektifiziersäule, die im Schritt (1) verwendet wird, zurückgeführt, zum Durchführen einer azeotropen Rektifizierung zusammen mit dem rohen Propylenoxidprodukt,

worin die erste azeotrope Rektifizierbedingung und die zweite azeotrope Rektifizierbedingung umfassen:

der Druck an der Säulenspitze der ersten azeotropen Rektifiziersäule ist 0,2 bis 0,8 MPa und der Druck an der Säulenspitze der zweiten azeotropen Rektifiziersäule ist 0,9 bis 1,5 MPa,
für die erste azeotrope Rektifiziersäule: die Temperatur am Säulenboden ist 100 bis 140°C, die Säulenplattenzahl ist 20 bis 45, das Rückflussverhältnis ist 2-5:1, die Zuführstelle ist unterhalb einer Stelle, die von dem Auslass der Säulenspitze um 1/4 des Abstandes von dem Auslass des Säulenbodens zu dem Auslass der Säulenspitze ist, und ist oberhalb einer Stelle, die von dem Auslass des Säulenbodens um 1/4 des Abstandes von dem Auslass des Säulenbodens zu dem Auslass der Säulenspitze entfernt ist, und ist zwischen dem Auslass des Säulenbodens und dem Auslass der Säulenspitze; für die zweite azeotrope Rektifiziersäule: die Temperatur am Säulenboden ist 110 bis 130°C, die Säulenplattenzahl ist 15 bis 25, das Rückflussverhältnis ist 0,8-3:1, die Zuführstelle ist unterhalb einer Stelle, die vom Auslass der Säulenspitze um 1/4 von dem Abstand von dem Auslass des Säulenbodens zu dem Auslass der Säulenspitze entfernt ist und oberhalb einer Stelle, die von dem Auslass des Säulenbodens um 1/4 des Abstandes im Auslass des Säulenbodens zu dem Auslasses der Säulenspitze entfernt ist.

2. Verfahren gemäß Anspruch 1, worin in dem rohen Propylenoxidprodukt der Gehalt von Propylenoxid 5 bis 99,5 Gew.%, der Gehalt von Methanol 0,5 bis 95 Gew.%, bezogen auf das Gesamtgewicht des rohen Propylenoxidproduktes, bezogen auf Methanol und Propylenoxid, ist.

3. Verfahren gemäß Anspruch 1, worin ein Ablass von dem Säulenboden der ersten azeotropen Rektifiziersäule einen Propylenoxidgehalt von weniger als 3 Gew.% hat und ein Ablass von dem Säulenboden der zweiten azeotropen Rektifiziersäule einen Methanolgehalt von weniger als 3 Gew.% hat.

4. Verfahren gemäß Anspruch 1, worin der erste azeotrope Rektifizierzustand und der zweite azeotrope Rektifizierzustand solche Zustände sind, dass der Gehalt an Methanol in dem Azeotrop, abgelassen von der Säulenspitze der ersten azeotropen Rektifiziersäule, weniger als der Gehalt von Methanol im Azeotrop ist, abgelassen von der Säulenspitze der zweiten azeotropen Rektifiziersäule.

5. Verfahren gemäß Anspruch 1, worin im Schritt (1) in der Mischung, die von der Säulenspitze der ersten azeotropen Rektifiziersäule abgelassen ist, die Gewichtskonzentration von Propylenoxid 90 bis 98 Gew.% und die Gewichtskonzentration von Methanol 2 bis 10 Gew.% ist; im Schritt (2) in der Mischung, die von der Säulenspitze der zweiten azeotropen Rektifiziersäule abgelassen ist, die Gewichtskonzentration von Propylenoxid 85 Gew.% bis weniger als 98 Gew.% ist und die Gewichtskonzentration von Methanol mehr als 2 bis 15 Gew.% ist.

6. Verfahren gemäß Anspruch 1, worin die erste azeotrope Rektifizierbedingung und die zweite azeotrope Rektifizierbedingung weiterhin umfassen: die Temperatur an der Säulenspitze der zweiten azeotropen Rektifiziersäule ist mehr als die Temperatur an der Säulenspitze der ersten azeotropen Rektifiziersäule.

7. Verfahren gemäß Anspruch 6, worin die erste azeotrope Rektifizierbedingung und die zweite azeotrope Rektifizierbedingung umfassen: die Temperatur an der Säulenspitze der ersten azeotropen Rektifiziersäule ist 60 bis 107°C, und die Temperatur an der Säulenspitze der zweiten azeotropen Rektifiziersäule ist 110 bis 125°C.

8. Verfahren gemäß einem der Ansprüche 1 bis 7, worin das rohe Propylenoxidprodukt erhalten wird durch ein Verfahren zur Herstellung von Propylenoxid, wobei das Verfahren das Kontaktieren von Propylen und $H_2O_2$ und die Reaktion unter einer Propylen-Epoxidierungsreaktionsbedingung in der Gegenwarte eines festen Katalysators umfasst und Methanol und wahlweise das restliche Propylen nach der Reaktion werden entfernt, zur Erzeugung des rohen Propylenoxidproduktes.

9. Verfahren gemäß Anspruch 8, worin die Reaktion von Propylen und $H_2O_2$ in der Gegenwart eines festen Katalysators und Methanol umfasst:

einen gemischten Strom, umfassend Methanol, Propylen und $H_2O_2$, wird aufeinanderfolgend in einen ersten und einen zweiten Reaktor geführt, worin der erste Reaktor ein Festbettreaktor oder ein Bewegbettreaktor ist und der zweite Reaktor ein Aufschlämmungsbettreaktor ist,

worin in dem ersten Reaktor die Propylen-Epoxidierungsreaktion so durchgeführt wird, dass die Umwandlungs-rate von $H_2O_2$ 50 bis 95 % erreicht; in dem zweiten Reaktor die Propylen-Epoxidierungsreaktion so durchgeführt wird, dass die gesamte Umwandlungsrate von $H_2O_2$ 98 % oder mehr erreicht, mit dem Vorbehalt, dass das Verfahren zur Herstellung von Propylenoxid eine Selektivität für Propylenoxid von 90 % oder mehr hat.

10. Verfahren gemäß Anspruch 9, worin
in dem Festbettreaktor die Propylen-Epoxidierungsreaktionsbedingung umfasst: die Temperatur ist 30 bis 90°C, der Druck ist 0,5 bis 4,5 MPa und die flüssige stündliche Volumenraumgeschwindigkeit des gemischten Stroms ist 1 bis 15 h$^{-1}$,
in dem Bewegbettreaktor umfasst die Propylen-Epoxidierungsreaktionsbedingung: die Temperatur ist 30 bis 90°C, der Druck 0,5 bis 4,5 MPa, die Reaktionszeit ist 0,2 bis 10 Stunden in Bezug auf 100 Gew.-Teile des gemischten Stromes, der in den Bewegbettreaktor gelangt, und enthält Methanol, Propylen und $H_2O_2$, die verwendete Menge des festen Katalyators ist 3 bis 10 Gew.-Teile;
im Aufschämmungsbettreaktor umfasst die Propylen-Epoxidierungsreaktionsbedingung: die Temperatur ist 30 bis 90°C, der Druck ist 0,5 bis 4,5 MPa, die Reaktionszeit ist 0,2 bis 10 Stunden, in Bezug auf 100 Gew.-Teile des Stroms, der von dem Festbettreaktor oder Bewegbettreaktor kommt und in den Aufschlämmungsbettreaktor kommt, die verwendete Menge des festen Katalysators ist 3 bis 10 Gew.-Teile.

11. Verfahren gemäß Anspruch 9, worin das Verfahren weiterhin enthält, bevor das resultierende Reaktionsprodukt, erhalten vom ersten Reaktor in den zweiten Reaktor gelangt, dass das resultierende Reaktionsprodukt, erhalten vom ersten Reaktor, einer Trennung unterworfen wird, unter Erhalt eines ersten Stromes, der frei ist von $H_2O_2$, und eines zweiten Stromes, der das nicht-reagierte $H_2O_2$ enthält, worin der erste Strom, der frei ist von $H_2O_2$, Propylen und Propylenoxid enthält, und der zweite Strom, der nicht-reagiertes $H_2O_2$ enthält, weiterhin das Lösungsmittel und Wasser enthält, Propylen zum zweiten Strom gegeben wird, und der zweite Strom, zu dem Propylen gegeben ist, in den zweiten Reaktor geführt wird, wahlweise das Gewichtsverhältnis von zu dem zweiten Strom gegebenem Propylen zu $H_2O_2$ in dem zweiten Strom 1-3:1 ist.

12. Verfahren gemäß Anspruch 9, worin das Verfahren weiterhin umfasst, bevor das resultierende Reaktionsprodukt, erhalten vom ersten Reaktor, in den zweiten Reaktor gelangt, dass Propylen zu dem Reaktionsprodukt geführt wird, das im ersten Reaktor erhalten wird, das Gewichtsverhältnis des zugeführten Propylens zu $H_2O_2$ in dem Reaktionsprodukt, erhalten vom ersten Reaktor, 1-2:1 ist.

13. Verfahren gemäß Anspruch 9, worin das molare Verhältnis von Methanol, Propylen und $H_2O_2$ im gemischten Strom 4-15:0,5-5:1 ist.

14. Verfahren gemäß Anspruch 9, worin der feste Katalysator ein Titansilikat ist.

15. Verfahren gemäß Anspruch 9, worin das restliche Propylen nach der Reaktion entfernt wird, so dass in dem resultierenden rohen Propylenoxidprodukt der Gehalt von Propylen weniger als 5 Gew.% ist, bezogen auf das Gewicht des rohen Propylenoxidproduktes.

**Revendications**

1. Procédé de raffinage d'un produit brut d'oxyde de propylène, dans lequel ledit produit brut d'oxyde de propylène est un mélange contenant de l'oxyde de propylène et du méthanol, procédé qui est **caractérisé en ce que** ledit procédé comprend :

(1) dans une première condition de rectification azéotropique, un produit brut d'oxyde de propylène est introduit dans une première colonne de rectification azéotropique afin de procéder à une rectification azéotropique, ladite première condition de rectification azéotropique est une condition telle que l'oxyde de propylène et le méthanol dans un courant introduit dans ladite première colonne de rectification azéotropique sont évacués du sommet de colonne en une forme d'azéotrope, et une partie résiduelle de méthanol et d'oxyde de propylène sont évacuées du fond de colonne, dans laquelle ladite partie résiduelle de méthanol évacuée du fond de colonne de ladite première colonne de rectification azéotropique a une pureté de 50 à 99 % en poids ; un effluent du fond de colonne de ladite première colonne de rectification azéotropique a une teneur en oxyde de propylène inférieure à 5 % en poids ;
(2) dans une deuxième condition de rectification azéotropique, un azéotrope évacué du sommet de colonne de ladite première colonne de rectification azéotropique à l'étape (1) est introduit dans une deuxième colonne de rectification azéotropique afin de procéder à une rectification azéotropique, ladite deuxième condition de rectification azéotropique est une condition telle que le méthanol et l'oxyde de propylène dans un mélange introduit dans ladite deuxième colonne de rectification azéotropique sont évacués du sommet de colonne de ladite deuxième colonne de rectification azéotropique sous forme d'azéotrope, une partie résiduelle de l'oxyde de propylène et du méthanol sont évacuées du fond de colonne de ladite deuxième colonne de rectification azéotropique, dans laquelle ladite partie résiduelle d'oxyde de propylène évacuée du fond de colonne de ladite deuxième colonne de rectification azéotropique a une pureté supérieure ou égale à 95 % en poids ; un effluent du fond de colonne de ladite deuxième colonne de rectification azéotropique a une teneur en méthanol inférieure à 5 % en poids ;

(3) éventuellement, l'azéotrope évacué du sommet de colonne de ladite deuxième colonne de rectification azéotropique à l'étape (2) est partiellement ou totalement renvoyé à ladite première colonne de rectification azéotropique utilisée à l'étape (1) afin de procéder à une rectification azéotropique conjointement avec le produit brut d'oxyde de propylène, dans laquelle ladite première condition de rectification azéotropique et ladite deuxième condition de rectification azéotropique comprennent :

la pression en tête de colonne de ladite première colonne de rectification azéotropique est de 0,2 à 0,8 MPa, et la pression en tête de colonne de ladite deuxième colonne de rectification azéotropique est de 0,9 à 1,5 MPa,

pour ladite première colonne de rectification azéotropique : la température au niveau du fond de colonne est de 100 à 140 °C ; le nombre de plaques de colonne est de 20 à 45, le rapport de reflux est de 2 à 5:1, l'emplacement de l'alimentation est au-dessous d'un emplacement qui est éloigné de la sortie du sommet de colonne d'un quart de la distance depuis la sortie du fond de colonne jusqu'à la sortie du sommet de colonne et au-dessus d'un emplacement qui est éloigné de la sortie du fond de colonne d'un quart de la distance depuis la sortie du fond de colonne jusqu'à la sortie du sommet de colonne, et est compris entre la sortie du fond de colonne et la sortie du sommet de colonne ; pour ladite deuxième colonne de rectification azéotropique : la température au niveau du fond de colonne est de 110 à 130 °C ; le nombre de plaques de colonne est de 15 à 25, le rapport de reflux est de 0,8 à 3:1, l'emplacement de l'alimentation est au-dessous d'un emplacement qui est éloigné de la sortie du sommet de colonne d'un quart de la distance depuis la sortie du fond de colonne jusqu'à la sortie du sommet de colonne et au-dessus d'un emplacement qui est éloigné de la sortie du fond de colonne d'un quart de la distance depuis la sortie du fond de colonne jusqu'à la sortie du sommet de colonne.

2. Procédé selon la revendication 1, dans lequel dans ledit produit brut d'oxyde de propylène, la teneur en oxyde de propylène est de 5 à 99,5 % en poids, la teneur en méthanol est de 0,5 à 95 % en poids, sur la base du poids total du produit brut d'oxyde de propylène contenant du méthanol et de l'oxyde de propylène.

3. Procédé selon la revendication 1, dans lequel un effluent du fond de colonne de ladite première colonne de rectification azéotropique a une teneur en oxyde de propylène inférieure à 3 % en poids, et un effluent du fond de colonne de ladite deuxième colonne de rectification azéotropique a une teneur en méthanol inférieure à 3 % en poids.

4. Procédé selon la revendication 1, dans lequel ladite première condition de rectification azéotropique et ladite deuxième condition de rectification azéotropique sont des conditions telles que la teneur en méthanol dans l'azéotrope évacué du sommet de colonne de ladite première colonne de rectification azéotropique est inférieure à la teneur en méthanol dans l'azéotrope évacué du sommet de colonne de ladite deuxième colonne de rectification azéotropique.

5. Procédé selon la revendication 1, dans lequel à l'étape (1), dans le mélange évacué du sommet de colonne de ladite première colonne de rectification azéotropique, la concentration en poids de l'oxyde de propylène est de 90 à 98 % en poids, et la concentration en poids du méthanol est de 2 à 10 % en poids ; à l'étape (2), dans le mélange évacué du sommet de colonne de ladite deuxième colonne de rectification azéotropique, la concentration en poids de l'oxyde de propylène est de 85 % en poids à moins de 98 % en poids, et la concentration en poids du méthanol est supérieure à 2 % en poids à 15 % en poids.

6. Procédé selon la revendication 1, dans lequel ladite première condition de rectification azéotropique et ladite deuxième condition de rectification azéotropique comprennent en outre : la température au niveau du sommet de colonne de ladite deuxième colonne de rectification azéotropique est supérieure à la température au niveau du sommet de colonne de ladite première colonne de rectification azéotropique.

**7.** Procédé selon la revendication 6, dans lequel ladite première condition de rectification azéotropique et ladite deuxième condition de rectification azéotropique comprennent : la température au niveau du sommet de colonne de ladite première colonne de rectification azéotropique est de 60 à 107 °C, et la température au niveau du sommet de colonne de ladite deuxième colonne de rectification azéotropique est de 110 à 125 °C.

**8.** Procédé selon l'une quelconque des revendications 1 à 7, dans lequel ledit produit brut d'oxyde de propylène est obtenu par un procédé de préparation d'oxyde de propylène, lequel procédé comprend la mise en contact et la réaction de propylène et d'$H_2O_2$ dans des conditions de réaction d'époxydation du propylène en présence d'un catalyseur solide et de méthanol et, éventuellement, l'élimination du propylène résiduel après la réaction pour produire ledit produit brut d'oxyde de propylène.

**9.** Procédé selon la revendication 8, dans lequel la réaction du propylène et de l'$H_2O_2$ en présence d'un catalyseur solide et de méthanol comprend
un courant mixte contenant du méthanol, du propylène et de l'$H_2O_2$ est chargé successivement dans un premier réacteur et un deuxième réacteur, dans lequel ledit premier réacteur est un réacteur à lit fixe ou un réacteur à lit mobile, et ledit deuxième réacteur est un réacteur à lit de boue liquide,
dans ledit premier réacteur, ladite réaction d'époxydation du propylène est effectuée de sorte que le taux de conversion de l'$H_2O_2$ atteint 50 % à 95 % ; dans ledit deuxième réacteur, ladite réaction d'époxydation du propylène est réalisée de sorte que le taux de conversion total de l'$H_2O_2$ atteint 98 % ou plus, à condition que ledit procédé de préparation d'oxyde de propylène ait une sélectivité pour l'oxyde de propylène de 90 % ou plus.

**10.** Procédé selon la revendication 9, dans lequel dans ledit réacteur à lit fixe, lesdites conditions de réaction d'époxydation du propylène comprennent : la température est de 30 à 90 °C, la pression est de 0,5 à 4,5 MPa, et la vitesse spatiale horaire en volume de liquide dudit courant mixte est de 1 à $15h^{-1}$ ;
dans ledit réacteur à lit mobile, lesdites conditions de réaction d'époxydation du propylène comprennent : la température est de 30 à 90 °C, la pression est de 0,5 à 4,5 MPa, le temps de réaction est de 0,2 à 10 heures, par rapport à 100 parties en poids du courant mixte entrant dans ledit réacteur à lit mobile et contenant du méthanol, du propylène et de l'$H_2O_2$, la quantité utilisée dudit catalyseur solide est de 3 à 10 parties en poids ;
dans ledit réacteur à lit de boue liquide, lesdites conditions de réaction d'époxydation du propylène comprennent : la température est de 30 à 90 °C, la pression est de 0,5 à 4,5 MPa, le temps de réaction est de 0,2 à 10 heures, par rapport à 100 parties en poids du courant entrant depuis ledit réacteur à lit fixe ou quittant ledit réacteur à lit mobile et entrant dans le réacteur à lit de boue liquide, la quantité utilisée dudit catalyseur solide est de 3 à 10 parties en poids.

**11.** Procédé selon la revendication 9, dans lequel ledit procédé comprend en outre avant que le produit de réaction résultant obtenu à partir du premier réacteur entre dans ledit deuxième réacteur, le produit de réaction résultant obtenu à partir du premier réacteur est soumis à une séparation pour obtenir un premier courant exempt d'$H_2O_2$ et un deuxième courant contenant l'$H_2O_2$ qui n'a pas réagi, dans lequel ledit premier courant exempt d'$H_2O_2$ contient du propylène et de l'oxyde de propylène, et ledit deuxième courant contenant l'$H_2O_2$ qui n'a pas réagi contient en outre le solvant et de l'eau ; le propylène est ajouté audit deuxième courant, et ledit deuxième courant auquel le propylène a été ajouté est chargé dans ledit deuxième réacteur, éventuellement, le rapport en poids du propylène ajouté audit deuxième courant d'$H_2O_2$ dans ledit deuxième courant est de 1 à 3:1.

**12.** Procédé selon la revendication 9, dans lequel ledit procédé comprend en outre avant que le produit de réaction résultant obtenu à partir du premier réacteur entre dans ledit deuxième réacteur, du propylène est supplémenté au produit réactionnel obtenu dans ledit premier réacteur, le rapport en poids du propylène supplémenté à l'$H_2O_2$ dans le produit de réaction obtenu à partir dudit premier réacteur est de 1 à 2:1.

**13.** Procédé selon la revendication 9, dans lequel le rapport molaire du méthanol, du propylène et de l'$H_2O_2$ dans ledit courant mixte est de 4 à 15:0,5 à 5:1.

**14.** Procédé selon la revendication 9, dans lequel ledit catalyseur solide est un silicate de titane.

**15.** Procédé selon la revendication 9, dans lequel le propylène résiduel est éliminé après la réaction de telle sorte que dans le produit brut d'oxyde de propylène obtenu, la teneur en propylène soit inférieure à 5 % en poids, par rapport au poids du produit brut d'oxyde de propylène.

Fig. 1

**REFERENCES CITED IN THE DESCRIPTION**

**Patent documents cited in the description**

- CN 1671678 A
- CN 1449392 A
- US 6500311 B1
- WO 2004092150 A
- CN 101279959 A
- CN 101274922 A